# EUROPEAN PATENT SPECIFICATION

(11) **EP 1 576 136 B1**
(45) Date of publication and mention of the grant of the patent: **25.05.2011**
(21) Application number: 03794659.7
(22) Date of filing: 05.09.2003
(51) Int. Cl.: C12Q 1/18, C12Q 1/68, A23J 3/34

(54) **METHODS FOR IDENTIFYING ANTIMICROBIAL COMPOUNDS**
VERFAHREN ZUR IDENTIFIZIERUNG ANTIMIKROBIELLER VERBINDUNGEN
METHODES D'IDENTIFICATION DE COMPOSES ANTIMICROBIENS

(30) Priority: 06.09.2002 US 409254
(43) Date of publication of application: 21.09.2005
(73) Proprietor: Novozymes, Inc., Davis, CA 95616 (US)
(72) Inventor: YAVER, Debbie, Davis, CA 95616 (US); SLOMA, Alan, Davis, CA 95616 (US)
(74) Representative: Kofoed, Gertrud Sonne
(86) International application number: PCT/US2003/028015
(87) International publication number: WO 2004/022720

(56) References cited:
- KUNST F ET AL: "THE COMPLETE GENOME SEQUENCE OF THE GRAM-POSITIVE BACTERIUM BACILLUS SUBTILIS" NATURE, NATURE PUBLISHING GROUP, LONDON, GB, vol. 390, 1997, pages 249-256, XP000919353 ISSN: 0028-0836
- YOSHIDA K-I ET AL: "Combined transcriptome and proteome analysis as a powerful approach to study genes under glucose repression in Bacillus subtilis" NUCLEIC ACIDS RESEARCH, OXFORD UNIVERSITY PRESS, SURREY, GB, vol. 29, no. 3, 1 February 2001 (2001-02-01), pages 683-692, XP002213298 ISSN: 0305-1048
- DUNMAN P M ET AL: "Transcription profiling-based identification of Staphylococcus aureus genes regulated by the agr and/or sarA loci" JOURNAL OF BACTERIOLOGY, vol. 183, no. 24, December 2001 (2001-12), pages 7341-7353, XP002445684 ISSN: 0021-9193
- RIMINI R ET AL: "Global analysis of transcription kinetics during competence development in Streptococcus pneumoniae using high-density DNA arrays" MOLECULAR MICROBIOLOGY, BLACKWELL SCIENTIFIC, OXFORD, GB, vol. 36, no. 6, June 2000 (2000-06), pages 1279-1292, XP002396766 ISSN: 0950-382X
- ULIJASZ A T ET AL: "A VANCOMYCIN-INDUCIBLE LACZ REPORTER SYSTEM IN BACILLUS SUBTILIS: INDUCTION BY ANTIBIOTICS THAT INHIBIT CELL WALL SYNTHESIS AND BY LYSOZYME" JOURNAL OF BACTERIOLOGY, WASHINGTON, DC, US, vol. 178, no. 21, November 1996 (1996-11), pages 6305-6309, XP001074640 ISSN: 0021-9193
- MOIR D T ET AL: "MICROARRAY DETECTION OF GENE EXPRESSION PROFILES IN STAPHYLOCOCCUS EPIDERMIDIS" ABSTRACTS OF THE INTERSCIENCE CONFERENCE ON ANTIMICROBIAL AGENTS AND CHEMOTHERAPY, vol. 41, 25 September 2001 (2001-09-25), page 249, XP008028401
- CHAN P E ET AL: "MICROARRAY GENE EXPRESSION PROFILING TO STUDY THE MODE OF ACTION OF DNA REPLICATION CLASS OF INHIBITORS IN STAPHYLOCOCCUS AUREUS" ABSTRACTS OF THE GENERAL MEETING OF THE AMERICAN SOCIETY FOR MICROBIOLOGY, vol. 102, 19 May 2002 (2002-05-19), page 9, XP008028290 ISSN: 1060-2011
- NG WAI-LEUNG ET AL: "Transcriptional regulation and signature patterns revealed by microarray analyses of Streptococcus pneumoniae R6 challenged with sublethal concentrations of translation inhibitors" JOURNAL OF BACTERIOLOGY, WASHINGTON, DC, US, vol. 185, no. 1, January 2003 (2003-01), pages 359-370, XP002312588 ISSN: 0021-9193
- ZHANG L. ET AL.: 'Regulated Gene Expression in S. aureus For Identifying Conditional Lethal Phenotypes and Antibiotic Mode of Action' GENE vol. 255, no. 2, July 2000, pages 297 - 305, XP004217644
- WILSON M. ET AL.: 'Exploring Drug-Induced Alterations In Gene Expression In Mycobacterium Tuberculosis By Microarray Hybridization' PNAS vol. 56, no. 22, October 1999, pages 12833 - 12838, XP002226713
- GMUENDER H. ET AL.: 'Gene Expression Changes Triggered by Exposure of H. influenza To Novobiocin or Ciprofloxacin: Combined Transcription and Translation Analysis' GENOME RES. vol. 11, 2001, pages 28 - 42, XP003003675

### SEQUENCE LISTING

<110> NOVOZYMES BIOTECH
<120> Methods For Identifying Markers Of Antimicrobial Compounds
<130> 10322.204-WO
<150> 10/243,698
   <151> 2002-09-06
<160> 6
<170> PatentIn version 3.2
<210> 1
   <211> 29
   <212> DNA
   <213> Bacillus subtilis
<400> 1
   cgggatccag cttgttgaaa aaccctcgc 29
<210> 2
   <211> 48
   <212> DNA
   <213> Bacillus subtilis
<400> 2
   tgctttcttt agtatcatca aagcttccgc ttccttggca gcgtgtgt 48
<210> 3
   <211> 48
   <212> DNA
   <213> Bacillus subtilis
<400> 3
   acacacgctg ccaaggaagc ggaagctttg atgatactaa agaaagca 48
<210> 4
   <211> 30
   <212> DNA
   <213> Bacillus subtilis
<400> 4
   ggggtaccag cgtgtaggca aaccttcgca 30
<210> 5
   <211> 26
   <212> DNA
   <213> Bacillus subtilis
<400> 5
   tagacaattg gaagagaaaa gagata 26
<210> 6
   <211> 26
   <212> DNA
   <213> Bacillus subtilis
<400> 6
   tagacaattg gaagagaaaa gagata 26

## Description

### Background of the Invention

### Field of the Invention

The present invention relates to methods for determining the mode of action of an antimicrobial compound. The present invention also relates to nucleic acid sequences which can be used as markers for antimicrobial drug screening.

### Description of the Related Art

Antimicrobial compounds are generally classified by their primary mode of action or mechanism. Such modes of action include inhibition of cell wall synthesis, cell membrane synthesis, protein synthesis, and nucleic acid synthesis. Other modes of action include interference with the cell membrane and competitive inhibition which primarily involves "growth factor analogs" that are structurally similar to a bacterial growth factor but which do not fulfill the metabolic function in the cell, *e.g.*, inhibition of folic acid biosynthesis. A review of the field of antimicrobial compounds and a description of the modes of action that form the basis for the classification system is found in "The Antimicrobial Drugs", Scholar, Eric M., and Pratt, William B., Oxford University Press, 2000, 2nd Edition.

The most common antimicrobial compounds are antibiotics. With the steady increase in antibiotic resistance to bacterial pathogens, there is a constant need for the development and discovery of new antibiotic compounds. Disease-causing microbes that have become resistant to antibiotic therapy are an increasing public health problem. Tuberculosis, gonorrhea, malaria, and childhood ear infections are just a few of the diseases that are increasingly difficult to treat with antibiotics. Part of the problem is that bacteria and other microorganisms that cause infections are remarkably resilient and can develop ways to survive drugs meant to kill or weaken them. Antibiotic resistance, also known as antimicrobial resistance or drug resistance, is also aided by the well-documented increase in the use of antibiotics in many fields and applications. It is estimated by the FDA that as much as 70 percent of the bacteria that cause infections in hospital settings are now resistant to at least one of the antibiotics commonly used to treat infections, and some organisms have become resistant to all approved antibiotics. For these extreme situations, patients must be treated with experimental and even potentially toxic drugs.

The discovery of new compounds for use as antimicrobial agents is hampered by several constraints. The first is the ability to screen large numbers of compounds for their potential as an antimicrobial drug, rather than just for bactericidal or bacteriostatic activity. In many cases, candidate compounds are synthetic drugs designed to mimic the structure and expected mechanism of a known antimicrobial compound. In other cases, however, even if the drug shows an apparent effect, the actual mode of action of the new drug may be difficult to assess. Further, even for promising new compounds, if they do not have an apparent structural relationship to a known compound, it can be difficult to determine the mode of action of the compound and predict whether the compound presents a likely therapeutic agent, or whether it is expected to show a broad, narrow or limited spectrum against bacteria. Without knowing the mode of action, it can be difficult to determine the likelihood that the compound will be selective, and hence less likely to produce toxic side-effects in a patient. Further, it is difficult to assay and identify from the countless potential antimicrobial compounds those which do not have an obvious known mode of action. However, the discovery of compounds which have a new target, or a different mode of action would also be desirable, as bacterial resistance strategies, once adapted, often apply to many or all of the compounds across a class. Compounds having a novel mode of action are a desired object of many research and discovery efforts in the antimicrobial field.

Morrow and Shaw, Pathogen Genomics: Impact on Human Health pp. 97-112 (Humana Press Inc., Totowa, New Jersey, 2002, disclose the use of DNA microarray expression analysis in antibacterial drug discovery.

GMUENDER H. et al. (Gene Expression Changes Triggered by Exposure of H. influenza To Novobiocin or Ciprofloxacin: Combined Transcription and Translation Analysis, GENOME RES. vol. 11, 2001, pages 28 - 42) discloses microarray expression analysis in *Haemophilus influenza* comparing two functional classes of DNA gyrase inhibitors (abstract, and pages 31-32), wherein low inhibitor concentrations are used, approximating the minimal inhibitory concentration (MIC; p. 32, top of left-hand column). This document raises also the possibility that at the higher concentration used (10X MIC), the concentration may be too high to determine the primary effect on the gene expression and the observations are mostly secondary effect (p. 32, bottom of right-hand column; p. 38, right-hand column "a low concentration, around the MIC values [...] seems to provide the best results for detection of the genes that are primarily affected.").

It is an object of the present invention to provide alternative methods for determining the mode of action of an antimicrobial compound. New marker genes in screening for new antimicrobial compounds are also provided.

### Summary of the Invention

The present invention relates to methods for determining the mode of action of an antimicrobial compound, comprising: (a) detecting hybridization complexes formed by contacting at least one nucleic acid sample, obtained by culturing *Bacillus subtilis* cells in the presence of at least one sub-inhibitory amount of an antimicrobial compound having an unknown mode of action, with a plurality of nucleic acid sequences corresponding to genes of the *Bacillus subtilis* cells, wherein the presence, absence or change in the amount of the hybridization complexes detected, compared with hybridization complexes formed between the plurality of nucleic acid sequences and a second nucleic acid sample obtained from the *Bacillus subtilis* cells cultured in the absence or presence of a standard compound having a known mode of action selected from the group consisting of cephalothin, chloramphenicol, ciprofloxacin, erythromycin, gentamicin, gramicidin A, nalidixic acid, norfloxacin, novobiocin, rifampin, streptomycin, trimethoprim, and vancomycin, is indicative of the similarity or dissimilarity of the mode of actions of the antimicrobial compound and the standard compound; and (b) assigning a mode of action for the antimicrobial compound based on the similarity or dissimilarity of values assigned to the hybridization complexes detected in (a) based on the relative amount of hybridization to a second set of hybridization values assigned to the hybridization complexes formed from the second nucleic acid sample; wherein the sub-inhibitory amount is based on the MIC of the antimicrobial compound against a bacterium and cultivation of the bacterium at one or more concentrations below the MIC. In a preferred embodiment, the method further comprises: (c) identifying from the plurality of nucleic acid sequences at least one sequence, or a homolog thereof, from the nucleic acid sample obtained from the *Bacillus subtilis* cells cultivated in the presence of the antimicrobial compound that has a detected expression level that is significantly different from the nucleic acid sample obtained from the *Bacillus subtilis* cells cultivated in the absence of the antimicrobial compound.

### Description of the Figures

Figure 1 shows a restriction map of pGME016.
Figure 2 shows a restriction map of pGME019.
Figure 3 shows a restriction map of pGME021.

### Detailed Description

The present invention relates to methods for determining the mode of action of an antimicrobial compound. The methods comprise: (a) detecting hybridization complexes formed by contacting at least one nucleic acid sample, obtained by culturing *Bacillus subtilis* cells in the presence of at least one sub-inhibitory amount of an antimicrobial compound having an unknown mode of action, with a plurality of nucleic acid sequences corresponding to genes of the *Bacillus subtilis* cells, wherein the presence, absence or change in the amount of the hybridization complexes detected, compared with hybridization complexes formed between the plurality of nucleic acid sequences and a second nucleic acid sample obtained from the *Bacillus subtilis* cells cultured in the absence or presence of a standard compound having a known mode of action selected from the group consisting of cephalothin, chloramphenicol, ciprofloxacin, erythromycin, gentamicin, gramicidin A, nalidixic acid, norfloxacin, novobiocin, rifampin, streptomycin, trimethoprim, and vancomycin, is indicative of the similarity or dissimilarity of the mode of actions of the antimicrobial compound and the standard compound; and (b) assigning a mode of action for the antimicrobial compound based on the similarity or dissimilarity of values assigned to the hybridization complexes detected in (a) based on the relative amount of hybridization to a second set of hybridization values assigned to the hybridization complexes formed from the second nucleic acid sample; wherein the sub-inhibitory amount is based on the MIC of the antimicrobial compound against a bacterium and cultivation of the bacterium at one or more concentrations below the MIC.

The methods of the present invention may be used to monitor global expression of a plurality of genes from a bacterial cell to identify genes which are primarily affected when the bacterial cell is subjected to an antimicrobial compound having an unknown mode of action. The expression profile generated by the treatment with the antimicrobial compound may then be compared to expression profiles obtained with compounds of a known mode of action to ascertain the mechanism of the antimicrobial compound. The gene expression patterns can also be used to identify and select nucleic acid molecules whose expression is either up-regulated or down-regulated due to the response to the antimicrobial compound. The genes primarily affected by the antimicrobial compound may then be selected and isolated and used as target or reporter genes for the development of new antimicrobial compounds. In addition, identification of genes secondarily affected by the primary effects of the antimicrobial compound may also be isolated and used as reporter genes for the development of new antimicrobial compounds. These selected molecules may then be employed as array elements alone or in combination with other array element molecules, employing methods well known to the art. The arrayed nucleic acid molecules are selected to optimize their performance in hybridization. Alternative formats known in the art may be used in place of arrays in the methods of the present invention where, for example, one gene or small set of genes, *e.g.*, an operon, is determined to be directly affected by an antimicrobial compound. Such alternative formats include, but are not limited to, Southern blots, slot blots, dot blots, and Northern blots.

The present methods are particularly advantageous because they utilize sub-inhibitory amounts of an antimicrobial compound to more readily identify primary effects of the antimicrobial compound on genes of the bacterial cell and reduce secondary effects on other genes that can result from using high inhibitor concentrations of the compound. The use of sub-inhibitory concentrations slows the action of the compounds, and limits the expression of genes which are correlated to secondary stress induced proteins, allowing a predominance of expressed nucleic acids which correlate with the activity of the antimicrobial compound which is related directly, and primarily, with its mode of action on the cell.

### Definitions

"Antimicrobial compound" or "antimicrobial agent" is any compound, molecule, or agent that elicits a biochemical, metabolic, and/or physiological response in bacteria that induces bacteriostasis or morbidity.
A "standard antimicrobial compound" or "standard compound" refers to an antimicrobial compound with a known mode of action.
"Sample" is used in its broadest sense herein. A sample containing nucleic acid molecules may include, but is not limited to, a cell; an extract from a cell, chromosome, organelle, or membrane isolated from a cell; genomic DNA, RNA, or cDNA in solution or bound to a substrate; a biological tissue or biopsy thereof; a fingerprint or tissue print; and natural or synthetic fibres; which is in a solution, liquid suspension, gaseous suspension, aerosol, and the like.
"Plurality" refers preferably to a group of one or more members, preferably to a group of at least about 10, more preferably to a group of at least about 100 members, even more preferably a group of 1,000 members, even more preferably at least about 5,000 members, and most preferably at least about 10,000 members.
"Substrate" refers to a rigid or semi-rigid support to which nucleic acid molecules or proteins are bound and includes membranes, filters, chips, slides, wafers, fibers, magnetic or nonmagnetic beads, gels, capillaries or other tubing, plates, polymers, and microparticles with a variety of surface forms including wells, trenches, pins, channels, and pores.
"Array" or "microarray" or "macroarray" refers to an ordered arrangement of hybridizable array elements on a substrate. The array elements are arranged so that there are preferably at least ten or more different array elements. In alternative embodiments, at least 100 array elements, even more preferably at least 1000 array elements, and most preferably 10,000 array elements are employed. The hybridization signal from each of the array elements is individually distinguishable. In a preferred embodiment, the array elements comprise nucleic acid molecules.
"Nucleic acid molecule" refers to a nucleic acid, oligonucleotide, nucleotide, polynucleotide or any fragment thereof. It may be DNA or RNA of genomic or synthetic origin, double-stranded or single-stranded, and combined with carbohydrate, lipids, protein, or other materials to perform a particular activity such as transformation or form a useful composition such as a peptide nucleic acid (PNA). "Oligonucleotide" is substantially equivalent to the terms primer, oligomer, element, target, and probe and is preferably single stranded.
"Up-regulated" refers to a nucleic acid molecule whose levels increase in a nucleic acid sample obtained by cultivating a bacterial strain in the presence of an antimicrobial compound compared with a nucleic acid sample from an untreated bacterial strain.
"Down-regulated" refers to nucleic acid molecule whose levels decrease in a nucleic acid sample obtained by cultivating a bacterial strain in the presence of an antimicrobial compound compared with a nucleic acid sample from an untreated bacterial strain.
The term "marker gene" or "marker" refers to a bacterial gene that is directly or indirectly affected by the action of an antimicrobial compound. A marker gene may be designated a target gene because it is directly affected by the compound. A marker gene may also be referred to as a reporter gene because it is either directly or indirectly affected by the antimicrobial compound.
"Fragment" refers to a part of a molecule which retains a usable, functional characteristic. Useful fragments include oligonucleotides and polynucleotides which may be used in hybridization or amplification technologies or in regulation of replication, transcription or translation.
"Hybridization complex" refers to a complex between two nucleic acid molecules by virtue of the formation of hydrogen bonds between purines and pyrimidines.

### Antimicrobial Compounds

In the methods of the present invention, the standard antimicrobial compound is selected from the group consisting of cephalothin, chloramphenicol, ciprofloxacin, erythromycin, gentamicin, gramicidin A, nalidixic acid, norfloxacin, novobiocin, rifampin, streptomycin, trimethoprim, and vancomycin.

The range of bacteria or other microorganisms that are affected by a certain antimicrobial compound is expressed as the spectrum of action. Antimicrobial compounds that kill or inhibit a wide range of Gram-positive and Gram-negative bacteria are said to be broad spectrum. If effective mainly against either Gram-positive or Gram-negative bacteria, they are narrow spectrum. If an antimicrobial compound is effective against a single organism or disease, it is referred to as having a limited spectrum.

Compounds in the class of cell wall synthesis inhibitors typically inhibit a step in the synthesis of bacterial peptidoglycan. Among this class are beta lactam antibiotics, which contain a 4-membered beta lactam ring. These compounds are the products of two groups of fungi, *Penicillium* and *Cephalosporium,* and are correspondingly represented by the penicillins and cephalosporins. The beta lactam antibiotics inhibit the last step in peptidoglycan synthesis, which involves the final cross-linking of peptide side chains, mediated by bacterial carboxypeptidase and transpeptidase enzymes. Beta lactam antibiotics are normally bactericidal and require that cells be actively growing in order to exert their toxicity.

Semisynthetic penicillins are compounds chemically-modified by the addition of side chains. Such compounds have been developed around disadvantages over natural penicillins, and will have an increased spectrum of activity (effectiveness against Gram-negative bacteria), resistance to penicillinase, and effectiveness when administered orally. Amoxicillin and ampicillin have broadened spectra against Gram-negative bacteria and are effective orally. Methicillin is penicillinase-resistant.

Cephalosporins are beta lactam antibiotics with a similar mode of action to penicillins, but which are produced by species of *Cephalosporium.* They have low toxicity and a somewhat broader spectrum than natural penicillins, and are often used as penicillin substitutes, against Gram-negative bacteria, and in surgical prophylaxis. They are subject to degradation by some bacterial beta-lactamases, but tend to be resistant to beta-lactamases from *Staphylococcus aureus.* Cephalothin is a first generation cephalosporin that has been in use longer than any other cephalosporins. Cepharparin and cephradine also belong to this class of compounds.

Of the cell wall synthesis inhibitors, natural penicillins, such as Penicillin G and Penicillin V, are produced by fermentation of *Penicillium chrysogenum.* They are effective against streptococcus, gonococcus and staphylococcus, except where resistance has developed. They are considered narrow spectrum since they are not effective against Gram-negative bacteria. Narrow spectrum penicillinase-resistant penicillins include methicillin, nafcillin, oxacillin, cloxacillin, and dicloxacillin.

Broad spectrum aminopenicillins include ampicillin, amoxicillin, proampicillins, carbenicillins, ticarcillin, and azlocillin.

Bacitracin is a polypeptide antibiotic produced by *Bacillus* species. It prevents cell wall growth by inhibiting the release of the muropeptide subunits of peptidoglycan from the lipid carrier molecule that carries the subunit to the outside of the membrane. Teichoic acid synthesis, which requires the same carrier, is also inhibited. Bacitracin has a high toxicity that precludes its systemic use.

Three additional synthetic chemotherapeutic agents that have been used in the treatment of tuberculosis are isoniazid (INH), paraaminosalicylic acid (PAS), and ethambutol. Ethambutol inhibits incorporation of mycolic acids into the mycobacterial cell wall. Isoniazid has been reported to inhibit mycolic acid synthesis in mycobacteria and since it is an analog of pyridoxine (Vitamin B6) it may inhibit pyridoxine catalyzed reactions as well. Isoniazid is activated by a mycobacterial peroxidase enzyme and destroys several targets in the cell. PAS is an anti-folate.

Antimicrobial compounds that interfere with the cell membrane, such as gramicidin, act by disorganizing the structure or inhibiting the function of bacterial membranes. Compounds that disorganize or disrupt the cell membranes rapidly kill bacteria. Because of the similarity of phospholipids membranes in bacterial and eukaryotic cells, however, most compounds of this nature lack the specificity required for use as a therapeutic agent. Polymyxins, produced by *Bacillus polymyxis*, are effective against Gram-negative bacteria, though they are usually limited to topical applications. Polymyxins bind to membrane phospholipids and thereby interfere with membrane function.

Protein synthesis inhibitors act by inhibiting translation at the level of the ribosome, binding the 30S and/or 50S subunits of the ribosomes, which provides the selective toxicity desired for an antimicrobial drug. The most important antibiotics with this mode of action are the tetracyclines, chloramphenicol, the macrolides (*e.g.,* erythromycin), and the aminoglycosides (*e.g.*, streptomycin).

The aminoglycosides are products of *Streptomyces* species and include streptomycin, kanamycin, tobramycin and gentamicin. These antibiotics exert their activity by binding to bacterial ribosomes and preventing the initiation of protein synthesis. Aminoglycosides have been used against a wide variety of bacterial infections caused by Gram-positive and Gram-negative bacteria.

The tetracyclines are antibiotics which are natural products of *Streptomyces,* although some are produced semisynthetically. Tetracycline, chlortetracycline, and doxycycline are the best known. The tetracyclines are broad spectrum antibiotics with a wide range of activity against both Gram-positive and Gram-negative bacteria. The tetracyclines act by blocking the binding of aminoacyl tRNA to the A site on the ribosome. Tetracyclines inhibit protein synthesis on isolated 70S or 80S (eukaryotic) ribosomes, and in both cases, their effect is on the small ribosomal subunit. However, most bacteria possess an active transport system for tetracycline that will allow intracellular accumulation of the antibiotic at concentrations 50 times as great as that in the medium. This greatly enhances its antibacterial effectiveness and accounts for its specificity of action, since an effective concentration cannot be accumulated in animal cells.

Chloramphenicol, originally purified from the fermentation of a *Streptomyces,* currently is produced by chemical synthesis. Chloramphenicol inhibits the bacterial enzyme peptidyl transferase thereby preventing the growth of the polypeptide chain during protein synthesis. Chloramphenicol is entirely selective for 70S ribosomes and does not affect 80S ribosomes.

The macrolides are a class of compounds that include erythromycin and oleandomycin. Erythromycin is active against most Gram-positive bacteria, *Neisseria*, *Legionella* and *Haemophilus*, but not against the Enterobacteriaceae. Macrolides inhibit bacterial protein synthesis by binding to the 50S ribosomal subunit. Macrolides are bacteriostatic for most bacteria but are bacteriacidal for a few Gram-positive bacteria.

Competitive inhibitors are mostly synthetic compounds. Most are "growth factor analogs" which are structurally similar to a bacterial growth factor but which do not fulfill the metabolic function in the cell. Some are bacteriostatic and some are bactericidal. The sulfonamides (*e.g.*, gantrisin), as well as trimethoprim, are inhibitors of the bacterial enzymes required for the synthesis of tetrahydrofolic acid (THF), the vitamin form of folic acid essential for 1-carbon transfer reactions. Sulfonamides are structurally similar to para-aminobenzoic acid (PABA), the substrate for the first enzyme in the THF pathway, and they competitively inhibit that step. Trimethoprim is structurally similar to dihydrofolate (DHF) and competitively inhibits the second step in THF synthesis mediated by DHF reductase. Animal cells do not synthesize their own folic acid but obtain it in a preformed fashion as a vitamin. Since animals do not make folic acid, they are not affected by these drugs, resulting in selective toxicity for bacteria.

Some chemotherapeutic agents affect the synthesis of DNA or RNA, or can bind to DNA or RNA so that their messages cannot be read, and for this reason, most of these agents are unselective. Two nucleic acid synthesis inhibitors which have selective activity against procaryotes and some medical utility are nalidixic acid and the rifamycins, *e.g.*, rifampin.

Nalidixic acid is a synthetic compound which has activity mainly against Gram-negative bacteria and belongs to a group of compounds called quinolones. Nalidixic acid is a bactericidal agent that inhibits DNA gyrase activity by binding to the DNA gyrase enzyme (topoisomerase), which is essential for DNA replication and allows supercoils to be relaxed and reformed. Nalidixic acid is effective against several types of Gram-negative bacteria such as *E. coli*, *Enterobacter aerogenes, Klebsiella pneumoniae,* and *Proteus* species. Gram-positive bacteria are resistant.

The rifamycins are also products of *Streptomyces.* Rifampicin is a semisynthetic derivative of rifamycin that is active against Gram-positive bacteria (including *Mycobacterium tuberculosis*) and some Gram-negative bacteria. Rifampicin acts specifically on eubacterial RNA polymerase and is inactive towards RNA polymerase from animal cells or towards DNA polymerase. The antibiotic binds to the beta subunit of the polymerase and is believed to prevent the entry of the first nucleotide which is necessary to activate the polymerase, thereby blocking mRNA synthesis.

In the methods of the present invention, the antimicrobial compound having an unknown mode of action may be any compound, molecule, or agent that elicits a biochemical, metabolic, and/or physiological response in bacteria that induces bacteriostasis or morbidity.

The antimicrobial compounds and minimal inhibitory concentration (MIC) may be known, or the compound and/or the MIC for the compound may be unknown. The determination of the MIC is well within the capability of those skilled in the art. MIC is defined as that concentration of an antimicrobial compound resulting in no visible growth of the organism.

### Bacterial Cells

In the methods of the present invention, the bacterium is Bacillus subtilis.

In the methods of the present invention, the bacterium is cultivated in a nutrient medium suitable for growth using methods well known in the art for isolation of the nucleic acids to be used as probes. For example, the cells may be cultivated by shake flask cultivation, small-scale or large-scale fermentation (including continuous, batch, fed-batch, or solid state fermentations) in laboratory or industrial fermentors performed in a suitable medium. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection).

The cultivation may also be a co-cultivation with mammalian cells to mimic an infection.

The bacterial cells are cultured in the absence and presence of at least one sub-inhibitory amount of an antimicrobial compound of interest. The sub-inhibitory amount is based on the MIC of the antimicrobial compound against a bacterium and cultivation of the bacterium at one or more concentrations below the MIC. Preferably, the sub-inhibitory amount is 0.5X MIC, more preferably 0.25X MIC, and most preferably 0.1X MIC.

### Nucleic Acid Samples

Nucleic acid samples are obtained by cultivating the bacterial cells at sub-inhibitory doses with one or more test antimicrobial compounds over a defined time course. The samples may be successive samples taken over a course of time and/or samples taken from cells cultured with two or more different sub-inhibitory concentrations of the compound. The nucleic acid samples from the bacterial cells, to be used as probes in forming hybridization complexes, may be any nucleic acid including genomic DNA, cDNA, RNA, peptide nucleic acids, branched DNAs, and the like, and may be isolated using standard methods known in the art. For example, cDNA probes may be obtained from the total mRNA isolated from the cells using standard methods and reverse transcribed into total cDNA.

The sample nucleic acids may be labeled with one or more labeling moieties to allow detection of the hybridized nucleic acid molecule complexes. The labeling moieties can include compositions that can be detected by spectroscopic, photochemical, biochemical, bioelectronic, immunochemical, electrical, optical, or chemical means. The labeling moieties include radioisotopes, such as ³²P, ³³P or ³⁵S, chemiluminescent compounds, labeled binding proteins, heavy metal atoms, spectroscopic markers, such as fluorescent markers and dyes, magnetic labels, linked enzymes, mass spectrometry tags, spin labels, electron transfer donors and acceptors, and the like using methods known in the art (see, for example, Chen et al., 1998, Genomics 51: 313-324; DeRisi et al., 1997, Science 278: 680-686; U.S. Patent No. 5,770,367).

In a preferred embodiment, the nucleic acid samples are labeled with fluorescent reporters. For example, cDNA samples may be labeled during reverse transcription from the respective mRNA pools by incorporation of fluorophores as dye-labeled nucleotides (DeRisi *et al.,* 1997, *supra*), *e.g.*, Cy5-labeled deoxyuridine triphosphate, or the isolated cDNAs may be directly labeled with different fluorescent functional groups. Fluorescent-labeled nucleotides include, but are not limited to, fluorescein conjugated nucleotide analogs (green fluorescence), lissamine nucleotide analogs (red fluorescence). Fluorescent functional groups include, but are not limited to, Cy3 (a green fluorescent dye) and Cy5 (red fluorescent dye).

### Nucleic Acid Sequences

In the methods of the present invention, the plurality of nucleic acid sequences corresponds to the genes of the bacterium. The term "correspond" is defined herein as nucleic acids that are identical or of sufficient homology to those in the bacterium. The term "sufficient homology" refers to the ability of the nucleic acids to cross-hybridize to the nucleic acids of the bacterium under conditions defined herein. The plurality of nucleic acid sequences corresponding to the genes of the bacterium may be of the same species, *i.e.*, identical or essentially identical, of the same genus but a different species, or of a different genus.

For purposes of the present invention, the degree of homology between two nucleic acid sequences is determined by the Wilbur-Lipman method (Wilbur and Lipman, 1983, Proceedings of the National Academy of Science USA 80: 726-730) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

In the methods of the present invention, the genes of the bacterium have a degree of homology to the plurality of nucleic acid sequences of at least 20%, preferably at least 40%, more preferably at least 60%, even more preferably at least 80%, even more preferably at least 90%, and most preferably at least 95% homology.

The plurality of sequences chosen for hybridization will depend on the genus or species, and whether the sequences are for broadly profiling the DNA expression in response to an antimicrobial compound, or are for assigning a mode of action by comparison to a known compound. The sequences may represent all or nearly all of the genome, or a portion of the genome. In other embodiments, the sequences may represent about 75% of the genome or less, about 50% of the genome or less, about 25% of the genome or less, about 10% of the genome or less, about 5% of the genome or less, or even about 2% of the genome or less.

The complete genome has been sequenced for a number of species of bacteria. The Comprehensive Microbial Resource (CMR) is a government funded initiative to encourage the sequencing of bacterial genomes, and to make the sequence information available to the community of researchers. The complete genomic sequences are made available through various publications, and the entire collection is maintained by The Institute for Genomic Research (TIGR). The CMR is fully described by Peterson et al., 2001, Nucleic Acids Research 29: 123-125.

Nucleic acids can be generated, for example, as follows: Total cellular DNA is isolated from a bacterial cell such as *Bacillus,* digested with a restriction endonuclease or cleaved by sonication, nebulization, or physical methods, size-selected by agarose gel electrophoresis, isolated, and ligated into a vector, *e.g.*, pSGMU2 (Errington, 1986, Journal of General Microbiology 132: 2953-2961). The ligation mixture is used to transform competent *E. coli* cells and transformants are selected under selective pressure, *e.g.*, ampicillin selection. Plasmids from the genomic DNA libraries are generated from random selected transformants, isolated, and partially sequenced. The partial sequences are then compared to sequences in various publicly available databases, for example GenBank, EMBL, Swissprot etc., for identification of function and annotated accordingly.

Any method known in the art may be used for generating nucleic acids (see, for example, Adams et al., 1991, Science 252: 1651-1656; Fields, 1996, Tibtech 14: 286-289; Weinstock et al., 1994, Current Opinion in Biotechnology 5: 599-603; Matsubara and Okubo, 1993, Current Opinions in Biotechnology 4: 672-677; Nelson et al., 1997, Fungal Genet. Biol. 21: 348-363; Roe *at al.*, http://www.genome.ou.edu/fungal.html).

In the methods of the present invention, the nucleic acids are preferably at least about 50 bp in length, more preferably at least about 100 bp in length, even more preferably at least about 150 bp in length, and most preferably at least about 200 bp in length. Furthermore, the nucleic acids are preferably directional nucleic acids. However, nondirectional nucleic acids may also be used. A "directional nucleic acid" is defined as a cDNA cloned in the same orientation relative to the vector cloning sites, *e.g.*, 5'→3'or 3'→5'.

In a preferred embodiment, the nucleic acids are obtained from *Bacillus subtilis.* In a more preferred embodiment, the nucleic acids are obtained from *Bacillus subtilis* strain BGSC1A2. In a most preferred embodiment, the nucleic acids are the genes or fragments thereof described by Kunst et al., 1997, Nature 390: 249-256.

In a preferred method, the plurality of nucleic acid molecules are those nucleic acid molecules that are different in samples treated with known antimicrobial compounds, or when treated with compounds from a particular class of compounds, as compared with nucleic acid molecules of untreated samples. Some of the nucleic acids may be up-regulated, or overexpressed, in the presence of the antimicrobial compound or molecule, and others may be down-regulated, or underexpressed. In other cases, expression of a gene may be absent in one sample, and present in the other. Still others may be up-regulated at one concentration, and down-regulated with another concentration of the compound.

In one aspect, the selected nucleic acid molecules which are up-regulated and/or down-regulated by a known antimicrobial compound or molecule are selected for use as the nucleic acids in the format of an array or any other suitable format known in the art such as Southern blots, zoo blots, slot blots, dot blots, and Northern blots.

### Microarrays

The term "an array of nucleic acids" is defined herein as a linear or two-dimensional array of preferably discrete elements of nucleic acids, each having a finite area, formed on the surface of a solid support.

The term "microarray" is defined herein as an array of nucleic acid elements having a density of discrete nucleic acid elements of at least about 100/cm², and preferably at least about 1000/cm². The nucleic acid elements in a microarray have typical dimensions, *e.g.*, diameters, in the range of between about 10 to about 250 µm, preferably in the range of between about 10 to about 200 µm, more preferably in the range of between about 20 to about 150 µm, even more preferably in the range of between about 20 to about 100 µm, most preferably in the range of between about 20 to about 75 µm, and even most preferably in the range of between about 25 to about 50 µm, and are separated from other nucleic acid elements in the microarray by about the same distance.

Methods and instruments for forming microarrays on the surface of a solid support are well known in the art. See, for example, U.S. Patent No. 5,807,522; U.S. Patent No. 5,700,637; and U.S. Patent No. 5,770,151. The instrument may be an automated device such as described in U.S. Patent No. 5,807,522. DNA glass spotted microarrays have also been used for bacterial expression studies (Schoolnik, et al., 2001, Methods Enzymol. 336: 3-18; Wilson, et al., 2001, Methods Mol. Med. 54: 335-358).

Any type of substrate known in the art may be used in the methods of the present invention. The term "a substrate containing an array of nucleic acids" is defined herein as a solid support having deposited on the surface of the support one or more of a plurality of nucleic acids for use in detecting binding of labeled DNAs to the nucleic acids.

The substrate may, in one aspect, be a glass support (*e.g.*, glass slide) having a hydrophilic or hydrophobic coating on the surface of the support, and an array of distinct nucleic acids electrostatically bound non-covalently to the coating, where each distinct nucleic acid is disposed at a separate, defined position.

Each microarray in the substrate preferably contains at least 10³ distinct nucleic acids in a surface area of less than about 1 cm². Each distinct nucleic acid (i) is disposed at a separate, defined position in the array, (ii) has a length of at least 50 bp, and (iii) is present in a defined amount between about 0.1 femtomoles and 100 nanomoles or higher if necessary.

For a hydrophilic coating, the glass slide is coated by placing a film of a polycationic polymer with a uniform thickness on the surface of the slide and drying the film to form a dried coating. The amount of polycationic polymer added should be sufficient to form at least a monolayer of polymers on the glass surface. The polymer film is bound to the surface via electrostatic binding between negative silyl-OH groups on the surface and charged cationic groups in the polymers. Such polycationic polymers include, but are not limited to, polylysine and polyarginine.

Another coating strategy employs reactive aldehydes to couple DNA to the slides (Schena et al., 1996, Proceedings of the National Academy of Science USA 93: 10614-10619; Heller et al., 1997, Proceedings of the National Academy of Science USA 94: 2150-2155).

Alternatively, the surface may have a relatively hydrophobic character, *i.e.*, one that causes aqueous medium deposited on the surface to bead. A variety of known hydrophobic polymers, such as polystyrene, polypropylene, or polyethylene, have desirable hydrophobic properties, as do glass and a variety of lubricant or other hydrophobic films that may be applied to the support surface. A support surface is "hydrophobic" if an aqueous droplet applied to the surface does not spread out substantially beyond the area size of the applied droplet, wherein the surface acts to prevent spreading of the droplet applied to the surface by hydrophobic interaction with the droplet.

In another aspect, the substrate may be a multi-cell substrate where each cell contains a microarray of nucleic acids, and preferably an identical microarray, formed on a porous surface. For example, a 96-cell array may typically have array dimensions between about 12 and 244 mm in width and 8 and 400 mm in length, with the cells in the array having width and length dimension of 1/12 and 1/8 the array width and length dimensions, respectively, *i.e.*, between about 1 and 20 in width and 1 and 50 mm in length.

High density oligonucleotide arrays, manufactured by Affymetrix, Inc., Santa Clara, CA, consist of 15 to 20 different 25-base oligonucleotides for each ORF of a sequenced genome; also represented in the same manner are intergenic regions greater than 200 bps (Lipshutz et al., 1999, Nat. Genet. 21: 20-24; Lockhart et al., 1996, Nat Biotechnol. 14: 1675-1649; Harrington et al., 2000, Curr. Opin. Microbiol. 3: 285-291). The selection of gene-specific oligonucleotides is based in part on sequence uniqueness in order to reduce cross-hybridization artifacts between paralogs, *i.e.*, other genes in the genome that contain related sequences. Each oligonucleotide is paired with a so-called "mismatch" control oligonucleotide that differs from its "perfect match" partner by only one, centrally-located base. Comparison of the hybridization intensity of the perfect match and mismatch oligonucleotide provides a method for determining and subtracting background fluorescence.

Membrane macroarrays also contain robotically-printed PCR products corresponding to each of the annotated ORFs of a genome. However, unlike the DNA glass-spotted microarrays described above, membrane macroarrays are produced by printing the double-strand amplicons onto positively-charged nylon membranes (Tao et al., 1999, J. Bacteriol. 181: 6425-6440).

The solid support may include a water-impermeable backing such as a glass slide or rigid polymer sheet, or other non-porous material. Formed on the surface of the backing is a water-permeable film which is formed of porous material. Such porous materials include, but are not limited to, nitrocellulose membrane nylon, polypropylene, and PVDF polymer. The thickness of the film is preferably between about 10 and 1000 µm. The film may be applied to the backing by spraying or coating, or by applying a preformed membrane to the backing.

The film surface may be partitioned into a desirable array of cells by water-impermeable grid lines typically at a distance of about 100 to 2000 µm above the film surface. The grid lines can be formed on the surface of the film by laying down an uncured flowable resin or elastomer solution in an array grid, allowing the material to infiltrate the porous film down to the backing, and then curing the grid lines to form the cell-array substrate.

The barrier material of the grid lines may be a flowable silicone, wax-based material, thermoset material (*e.g.*, epoxy), or any other useful material. The grid lines may be applied to the solid support using a narrow syringe, printing techniques, heat-seal stamping, or any other useful method known in the art.

Each well preferably contains a microarray of distinct nucleic acids. "Distinct nucleic acids" as applied to the nucleic acids forming a microarray is defined herein as an array member that is distinct from other array members on the basis of a different nucleic acid sequence, and/or different concentrations of the same or distinct nucleic acids, and/or different mixtures of distinct nucleic acids or different-concentrations of nucleic acids. Thus an array of "distinct nucleic acids" may be an array containing, as its members, (i) distinct nucleic acids, which may have a defined amount in each member, (ii) different, graded concentrations of given-sequence nucleic acids, and/or (iii) different-composition mixtures of two or more distinct nucleic acids.

The delivery of a known amount of a selected nucleic acid to a specific position on the support surface is preferably performed with a dispensing device equipped with one or more tips for insuring reproducible deposition and location of the nucleic acids and for preparing multiple arrays. Any dispensing device known in the art may be used in the methods of the present invention. See, for example, U.S. Patent No. 5,807,522. The dispensing device preferably contains a plurality of tips.

For liquid-dispensing on a hydrophilic surface, the liquid will have less of a tendency to bead, and the dispensed volume will be more sensitive to the total dwell time of the dispenser tip in the immediate vicinity of the support surface.

For liquid-dispensing on a hydrophobic surface, flow of fluid from the tip onto the support surface will continue from the dispenser onto the support surface until it forms a liquid bead. At a given bead size, *i.e.*, volume, the tendency of liquid to flow onto the surface will be balanced by the hydrophobic surface interaction of the bead with the support surface, which acts to limit the total bead area on the surface, and by the surface tension of the droplet, which tends toward a given bead curvature. At this point, a given bead volume will have formed, and continued contact of the dispenser tip with the bead, as the dispenser tip is being withdrawn, will have little or no effect on bead volume.

The desired deposition volume, *i.e.*, bead volume, formed is preferably in the range 2 pl (picoliters) to 2 nl (nanoliters), although volumes as high as 100 nl or more may be dispensed. It will be appreciated that the selected dispensed volume will depend on (i) the "footprint" of the dispenser tip(s), *i.e.*, the size of the area spanned by the tip(s), (ii) the hydrophobicity of the support surface, and (iii) the time of contact with and rate of withdrawal of the tip(s) from the support surface. In addition, bead size may be reduced by increasing the viscosity of the medium, effectively reducing the flow time of liquid from the dispensing device onto the support surface. The drop size may be further constrained by depositing the drop in a hydrophilic region surrounded by a hydrophobic grid pattern on the support surface.

At a given tip size, bead volume can be reduced in a controlled fashion by increasing surface hydrophobicity, reducing time of contact of the tip with the surface, increasing rate of movement of the tip away from the surface, and/or increasing the viscosity of the medium. Once these parameters are fixed, a selected deposition volume in the desired pl to nl range can be achieved in a repeatable fashion.

After depositing a liquid droplet of an nucleic acid sample at one selected location on a support, the tip may be moved to a corresponding position on a second support, the nucleic acid sample is deposited at that position, and this process is repeated until the nucleic acid sample has been deposited at a selected position on a plurality of supports.

This deposition process may then be repeated with another nucleic acid sample at another microarray position on each of the supports.

The diameter of each nucleic acid region is preferably between about 20-200 µm. The spacing between each region and its closest (non-diagonal) neighbor, measured from center-to-center, is preferably in the range of about 20-400 µm. Thus, for example, an array having a center-to-center spacing of about 250 µm contains about 40 regions/cm² or 1,600 regions/cm². After formation of the array, the support is treated to evaporate the liquid of the droplet forming each region, to leave a desired array of dried, relatively flat nucleic acid regions. This drying may be done by heating or under vacuum. The DNA can also be UV-crosslinked to the polymer coating.

### Hybridization

The labeled nucleic acids from the bacterial cells are added to a substrate containing an array of one or more nucleic acids, or other format as described herein, under conditions where the nucleic acid samples from the bacterial cells hybridize to complementary sequences of the nucleic acids in the array. For purposes of the present invention, hybridization indicates that the labeled nucleic acids from the bacterial cells hybridize to the nucleic acids on the array or other format under very low to very high stringency conditions.

For nucleic acid probes of at least about 100 nucleotides in length, very low to very high stringency conditions are defined as prehybridization and hybridization at 42°C in 5X SSPE, 0.3% SDS, 200 µg/ml sheared and denatured salmon sperm DNA, and either 25% formamide for very low and low stringencies, 35% formamide for medium and medium-high stringencies, or 50% formamide for high and very high stringencies, following standard Southern blotting procedures.

For nucleic acid probes of at least about 100 nucleotides in length, the carrier material is finally washed three times each for 15 minutes using 2X SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For shorter nucleic acid probes which are about 50 nucleotides to about 100 nucleotides in length, stringency conditions are defined as prehybridization, hybridization, and washing post-hybridization at 5°C to 10°C below the calculated Tₘ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For shorter nucleic acid probes which are about 50 nucleotides to about 100 nucleotides in length, the carrier material is washed once in 6X SCC plus 0.1 % SDS for 15 minutes and twice each for 15 minutes using 6X SSC at 5°C to 10°C below the calculated Tₘ.

The choice of hybridization conditions will depend on the degree of homology between the bacterial nucleic acids and the nucleic acids obtained from the two or more bacterial cells. For example, where the cells are the same cell from which the nucleic acids were obtained, high stringency conditions may be most suitable. Where the cells are from a genus or species different from which the nucleic acids were obtained, low or medium stringency conditions may be more suitable.

In a preferred embodiment, the hybridization is conducted under low stringency conditions. In a more preferred embodiment, the hybridization is conducted under medium stringency conditions. In a most preferred embodiment, the hybridization is conducted under high stringency conditions.

The entire solid support is then reacted with detection reagents if needed and analyzed using standard photometric, calorimetric, radioactive, or fluorescent detection means. All processing and detection steps are performed simultaneously to all of the microarrays on the solid support ensuring uniform assay conditions for all of the microarrays on the solid support.

### Detection

The most common detection method is laser-induced fluorescence detection using confocal optics (Cheung et al., 1998, Nat. Genet. 18: 225-230). The array is examined under fluorescence excitation conditions such that (i) the nucleic acids in the array that hybridize to the nucleic acid probes obtained from bacterial cells produce a distinct first fluorescence emission color or one or second fluorescence emission colors, respectively, and (ii) the nucleic acids in the array that hybridize to substantially equal numbers of nucleic acid probes obtained from bacterial cells produce a distinct combined fluorescence emission color, respectively; wherein the relative expression of the genes in the bacterial cells can be determined by the observed fluorescence emission color of each spot in the array.

The fluorescence excitation conditions are based on the selection of the fluorescence reporters. For example, Cy3 and Cy5 reporters are detected with solid state lasers operating at 532 nm and 632 nm, respectively.

However, other methods of detection well known in the art may be used such as standard photometric, calorimetric, or radioactive detection means.

In a differential hybridization experiment, nucleic acid molecules from two or more different biological samples are labeled with two or more different fluorescent labels with different emission wavelengths. Fluorescent signals are detected separately with different photomultipliers set to detect specific wavelengths. The relative abundances/expression levels of the nucleic acid molecules in two or more samples is obtained.

Typically, microarray fluorescence intensities can be normalized to take into account variations in hybridization intensities when more than one microarray is used under similar test conditions. In a preferred embodiment, individual arrayed-sample nucleic acid molecule complex hybridization intensities are normalized using the intensities derived from internal normalization controls contained on each microarray.

### Data Analysis

The data obtained from the scanned image may then be analyzed using any of the commercially available image analysis software. The software preferably identifies array elements, subtracts backgrounds, deconvolutes multi-color images, flags or removes artifacts, verifies that controls have performed properly, and normalizes the signals (Chen et al., 1997, Journal of Biomedical Optics 2: 364-374).

Several computational methods have been described for the analysis and interpretation of microarray-based expression profiles including cluster analysis (Eisen et al., 1998, Proc. Nat. Acad. Sci. USA 95: 14863-14868), parametric ordering of genes (Spellman et al., 1998, Mol. Biol. Cell 9: 3273-3297), and supervised clustering methods based on representative hand-picked or computer-generated expression profiles (Chu et al., 1998. Science 282: 699-705). Preferred methods for evaluating the results of the microarrays employ statistical analysis to determine the significance of the differences in expression levels. In the methods of the present invention, the difference in the detected expression level is at least about 10% or greater, preferably at least about 20% or greater, more preferably at least about 50% or greater, even more preferably at least about 75% or greater; and most preferably at least about 100% or greater.

One such preferred system is the Significance Analysis of Microarrays (SAM) (Tushe et al., 2001, Proc. Natl. Acad. Sci. USA 98: 5116-5121). Statistical analysis allows the determination of significantly altered expression of levels of about 50% or even less. The PAM (or predictive analysis for microarrays), represents another approach for analyzing the results of the microarrays (Tibshirani et al., 2002, Proc. Natl. Acad. Sci. USA 99: 6567-6572).

Cluster algorithms may also be used to analyze microarray expression data. From the analysis of the expression profiles it is possible to identify co-regulated genes that perform common metabolic or biosynthetic functions. Hierarchical clustering has been employed in the analysis of microarray expression data in order to place genes into clusters based on sharing similar patterns of expression (Eisen *et al.*, 1998, *supra*). This method yields a graphical display that resembles a kind of phylogenetic tree where the relatedness of the expression behavior of each gene to every other gene is depicted by branch lengths. The programs Cluster and TreeView, both written by Michael Eisen at Stanford University, are available at http://rana.stanford.edu/software/. Genespring is a commercial program available for such analysis.

Self-organizing maps (SOMs), a non-hierarchical method, have also been used to analyze microarray expression data (Tamayo et al., 1999, Proc. Natl. Acad. Sci. USA 96: 2907-2912). This method involves selecting a geometry of nodes, where the number of nodes defines the number of clusters. Then, the number of genes analyzed and the number of experimental conditions that were used to provide the expression values of these genes are subjected to an iterative process (20,000 - 50,000 iterations) that maps the nodes and data points into multidimensional gene expression space. After the identification of significantly regulated genes, the expression level of each gene is normalized across experiments. As a result, the expression profile of the genome is highlighted in a manner that is relatively independent of each gene's expression magnitude. Software for the "GENECLUSTER" SOM program for microarray expression analysis can be obtained from the Whitehead/MIT Center for Genome Research. SOMs can also be construcuted using the GeneSpring software package.

The methods of the present invention may be used for determining a mode of action for an antimicrobial compound, by comparison with hybridization with a second nucleic acid sample obtained from the bacterial cells cultured in the absence or presence of a standard compound having a known mode of action. In this method, the degree of similarity of the expression profiles is indicative of the similarity or dissimilarity of the mode of actions of the test compound and a known compound.

In a preferred embodiment, values are assigned to the hybridization complexes based on the relative amount of hybridization and the values are analyzed for the similarity or dissimilarity of the values to a second set of hybridization values assigned to the hybridization complexes formed from the second nucleic acid sample. A program for analysis, such as a computer algorithm, may be used to assign a mode of action for the test compound based on the degrees of similarity in the hybridization complexes.

When comparing the actions of different antimicrobial compounds, a similarity in the expression profile may mean that at least 1, preferably at least 5, more preferably at least 10, of the up-regulated arrayed genes commonly form hybridization complexes with the sample nucleic acid molecules at least once during a time course to a greater extent than would the nucleic acid molecules of a sample not treated with the test compound. Similarity may also mean that at least 1, preferably at least 5, more preferably at least 10, of the down-regulated nucleic acid molecules commonly form hybridization complexes with the arrayed genes at least once during a time course to a lesser extent than would the nucleic acid molecules of a sample not treated with the test compound or a known toxic compound.

A similarity of expression patterns indicates that the compounds have a similar mode of action on the bacterium.

### Marker Genes

The presence, absence or change in the amount of the hybridization complexes detected, as the case may be, based on a comparison of hybridization complexes formed with the same plurality of nucleic acid sequences and a nucleic acid sample obtained from untreated bacterial cells, provides a profile of genes expressed in the bacterial cells treated with an antimicrobial compound.

Some of the marker nucleic acids may be up-regulated, or overexpressed, in the presence of the antimicrobial compound, and others may be down-regulated, or underexpressed. In other cases, expression of a gene may be absent in one sample, and present in the other. Still others may be up-regulated at one concentration, and down-regulated with another concentration of the compound.

The present disclosure also relates to a sequence or sequences that are expressed in a unique fashion for a particular compound or class of antimicrobial compounds, or the signature of expression for that compound or class of antimicrobial compounds. In the methods of the present invention, the sequences are identified from the plurality of nucleic acid sequences, as sequences that form relative hybridization complexes that are significantly different from a nucleic acid sample obtained from untreated bacterial cells. The open reading frame, or ORF, can be identified in this method by relating the sequences of the microarray to the corresponding bacterial genes.

The present disclosure also includes arrays comprising a plurality of nucleic acid sequences that hybridize to nucleic acid sequences expressed in a bacteria treated with a sub-inhibitory amount of an antimicrobial compound, wherein the expressed nucleic acid sequences comprise a selection of nucleic acid sequences that are expressed in bacteria treated by antimicrobial compounds of a similar class, *i.e.*, compounds having a similar mode of action on a cell.

Once specific sequences or the expression profiles of sequences have been identified that are expressed in a bacterial cell exposed to a compound of a certain class of antimicrobial compounds, a novel compound can be evaluated for antimicrobial activity by testing the compound for the inhibition, interaction or interference with the normal expression or activity of the correlated bacterial gene. This can be done using an array or any oether suitable format known in the art, which has been created using sequences of the expression profile.

The nucleic acid sequences described, and fragments thereof, can, therefore, be used in various hybridization technologies involving microarrays, macroarrays, or other formats, as described herein. Hybridization sequences may be produced using oligolabeling, nick translation, end-labeling, or PCR amplification in the presence of the labeled nucleotide.

The nucleic acid sequences are preferably contained on a substrate. In a preferred embodiment, the substrate comprises the plurality of nucleic acid sequences selected from the group of genes of Tables 4-23 or fragments thereof.

In a preferred embodiment, the nucleic acid molecules comprising the plurality of nucleic acid sequences are selected sequences of the genes of Table 4, or fragments thereof.

In another preferred embodiment, the nucleic acid molecules comprising the plurality of nucleic acid sequences are selected sequences of the genes of Table 5, or fragments thereof.

In another preferred embodiment, the nucleic acid molecules comprising the plurality of nucleic acid sequences are selected sequences of the genes of Table 6, or fragments thereof.

In another preferred embodiment, the nucleic acid molecules comprising the plurality of nucleic acid sequences are selected sequences of the genes of Table 7, or fragments thereof.

In another preferred embodiment, the nucleic acid molecules comprising the plurality of nucleic acid sequences are selected sequences of the genes of Table 8, or fragments thereof.

In another preferred embodiment, the nucleic acid molecules comprising the plurality of nucleic acid sequences are selected sequences of the genes of Table 9, or fragments thereof.

In another preferred embodiment, the nucleic acid molecules comprising the plurality of nucleic acid sequences are selected sequences of the genes of Table 10, or fragments thereof.

In another preferred embodiment, the nucleic acid molecules comprising the plurality of nucleic acid sequences are selected sequences of the genes of Table 11, or fragments thereof.

In another preferred embodiment, the nucleic acid molecules comprising the plurality of nucleic acid sequences are selected sequences of the genes of Table 12, or fragments thereof.

In another preferred embodiment, the nucleic acid molecules comprising the plurality of nucleic acid sequences are selected sequences of the genes of Table 13, or fragments thereof.

In another preferred embodiment, the nucleic acid molecules comprising the plurality of nucleic acid sequences are selected sequences of the genes of Table 14, or fragments thereof.

In another preferred embodiment, the nucleic acid molecules comprising the plurality of nucleic acid sequences are selected sequences of the genes of Table 15, or fragments thereof.

In another preferred embodiment, the nucleic acid molecules comprising the plurality of nucleic acid sequences are selected sequences of the genes of Table 16, or fragments thereof.

In another preferred embodiment, the nucleic acid molecules comprising the plurality of nucleic acid sequences are selected sequences of the genes of Table 17, or fragments thereof.

In another preferred embodiment, the nucleic acid molecules comprising the plurality of nucleic acid sequences are selected sequences of the genes of Table 18, or fragments thereof.

In another preferred embodiment, the nucleic acid molecules comprising the plurality of nucleic acid sequences are selected sequences of the genes of Table 19, or fragments thereof.

In another preferred embodiment, the nucleic acid molecules comprising the plurality of nucleic acid sequences are selected sequences of the genes of Table 20, or fragments thereof.

In another preferred embodiment, the nucleic acid molecules comprising the plurality of nucleic acid sequences are selected sequences of the genes of Table 21, or fragments thereof.

In a more preferred embodiment, the nucleic acid molecules comprising the plurality of nucleic acid sequences are selected sequences of the *Bacillus subtilis* genes of Tables 22 and 23, or fragments thereof.

In another more preferred embodiment, the nucleic acid molecules comprising the plurality of nucleic acid sequences are selected sequences of the *Staphylococcus* genes of Table 22, or fragments thereof.

In another more preferred embodiment, the nucleic acid molecules comprising the plurality of nucleic acid sequences are selected sequences of the *Streptococcus* genes of Table 22, or fragments thereof.

In an even more preferred embodiment, the plurality of nucleic acid sequences is a marker for the mode of action of topoisomerase activity inhibition selected from the group of genes of Tables 4, 5, 6 or 7, or fragments thereof. In another even more preferred embodiment, the plurality of nucleic acid sequences is a marker for the mode of action of cell wall inhibitors selected from the group genes of Tables 8, 9, and 10, or fragments thereof. In another even more preferred embodiment, the plurality of nucleic acid sequences is a marker for the mode of action of protein synthesis inhibitors selected from the group of Tables 11-20. In another even more preferred embodiment, the plurality of nucleic acid sequences is a marker for the mode of action of RNA synthesis inhibitors selected from the group of Table 21.

In a most preferred embodiment, the plurality of nucleic acid sequences includes *yerQ* or a fragment thereof. In another most preferred embodiment, the plurality of nucleic acid sequences includes SA0681, or a fragment thereof, which is a marker for the mode of action of topoisomerase activity inhibition. In a most preferred embodiment, the plurality of nucleic acid sequences includes SA1714, or a fragment thereof, which is a marker for the mode of action of topoisomerase activity inhibition. In another most preferred embodiment, the plurality of nucleic acid sequences includes SP1045, or a fragment thereof, which is a marker for the mode of action of topoisomerase activity inhibition.

Alternatively, a promoter from the selected sequences may be utilized to produce a construct for insertion into the bacterial genome. Such a construct preferably includes a reporter gene, so that the treatment of the bacterial cell by an antimicrobial compound will trigger the promoter to express the reporter gene and signal the type of compound, or the mode of action for the compound.

Where a gene has been identified which is specifically expressed in response to an antimicrobial compound of a particular class, the promoter region for that gene can be used in a further embodiment of the invention, for determining a mode of action for an antimicrobial compound or for screening a library of compounds to find a molecule that gives a similar response. In this method, bacterial cells are treated with a test compound, where the bacterial cells comprise the responsive promoter linked to a reporter gene. By monitoring expression of the reporter gene, its presence, absence or a change in the amount of the expression of the reporter gene will be indicative of the similarity or dissimilarity of the mode of actions of the test compound and an antimicrobial compound of the known class of antimicrobial compounds for the responsive gene. Preferably, the responsive promoter is a promoter for a gene for which expression is induced in a cell when treated by an antimicrobial compound of a first class of antimicrobial compounds, but not by an antimicrobial compound of a second class of antimicrobial compounds.

A preferred method involves high-throughput screening of antimicrobial compounds utilizing such responsive promoters. In one such preferred method the responsive promoter construct is transformed into a *Bacillus* cell, and the *Bacillus* cell is treated with the antimicrobial compound. In a further preferred embodiment, a plurality of *Bacillus* cells are used, wherein each cell has been transformed by a responsive promoter construct, and wherein the pattern of expression of the marker genes is indicative of the mode of action for the antimicrobial compound.

Suitable reporter genes may include a gene expressing green fluorescent protein, luciferase, or β-galactosidase, or any of a number of drug resistance genes. The reporter gene may also be a protein which is detected by immunological screening.

The nucleic acid molecules may also be used to construct microarrays, where the DNA of the microarray has been selected to be enriched for genes which are uniquely expressed, either individually or collectively, in response to compounds having a particular mode of action. After hybridization, the microarray is washed to remove nonhybridized nucleic acid molecules and complex formation between the hybridizable array elements and the nucleic acid molecules is detected. Methods for detecting complex formation are well known to those skilled in the art. In a preferred embodiment, the nucleic acid molecules are labeled with a fluorescent label and measurement of levels and patterns of fluorescence indicative of complex formation is accomplished by fluorescence microscopy, preferably confocal fluorescence microscopy.

Furthermore, a gene identified by the methods of the present invention may be a target for further drug discovery, by testing for essential activity of the expression of the bacterial gene by various methods well known to those of ordinary skill in the art. Such methods may include preparing the gene as a knockout, repressing or inducing the activity of the gene, or mutagenizing the gene to alter its expression using methods well known in the art.

### Computer Readable Media

The marker nucleic acids described herein may be "provided" in a variety of media to facilitate their use. The term "provided" refers to a manufacture comprising the marker nucleic acids. Such manufactures are in a form which allows one skilled in the art to examine the manufacture using means not directly applicable to examining the genome or a subset thereof as it exists in nature or in purified form.

Thus, the present disclosure also relates to such a manufacture in the form of a computer readable medium comprising the marker nucleic acids.

In one application of this embodiment, the marker nucleic acids of the present invention can be recorded on computer readable media. The term "computer readable media" is defined herein as any medium which can be read and accessed directly by a computer. Such computer readable media include, but are not limited to, magnetic storage media, *e.g.*, floppy discs, hard disc storage medium, and magnetic tape; optical storage media, *e.g.*, CD-ROM, DVD; electrical storage media, *e.g.*, RAM and ROM; and hybrids of these categories, *e.g.*, magnetic/optical storage media. One skilled in the art can readily appreciate how any of the presently known computer readable media can be used to create a manufacture comprising computer readable media having recorded thereon one or more nucleotide sequences of the present invention. Likewise, it will be clear to those of skill how additional computer readable media that may be developed can also be used to create analogous manufactures having recorded thereon nucleotide sequence information of the present invention.

As used herein, "recorded" refers to a process for storing information on computer readable medium. One skilled in the art can readily adopt any of the presently known methods for recording information on computer readable medium to generate manufactures comprising the nucleotide sequence information of the present invention.

A variety of data storage structures are available for creating a computer readable medium having recorded thereon the marker nucleic acids of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. A skilled artisan can readily adapt any number of data-processor structuring formats (*e.g.*, text file or database) in order to obtain computer readable medium having recorded thereon the nucleotide sequence information of the present invention.

Various computer software are publicly available that allow a skilled artisan to access sequence information provided in a computer readable medium. Thus, by providing in computer readable form the marker nucleic acids, this enables one skilled in the art to routinely access the provided sequence information for a wide variety of purposes.

The term "a computer-based system" is defined herein as a hardware means, software means, and data storage means used to analyze the nucleotide sequence information of the present disclosure. The minimum hardware means of the computer-based systems of the present disclosure comprises a central processing unit (CPU), input means, output means, and data storage means. One skilled in the art can readily appreciate that any currently available computer-based system is suitable for use in the present disclosure.

As stated above, the computer-based systems described comprise a data storage means having stored therein marker nucleic acids of the present invention and the necessary hardware means and software means for supporting and implementing a search means.

The term "data storage means" is defined herein as memory which can store the nucleotide sequence information of the present disclosure, or a memory access means which can access manufactures having recorded thereon the nucleotide sequence information of the present disclosure.

The term "search means" is defined herein as one or more programs which are implemented on the computer-based system to compare a target sequence or target structural motif with the sequence information stored within the data storage means. A variety of known algorithms are disclosed publicly and a variety of commercially available software for conducting search means are and can be used in the computer-based systems described. Examples of such software includes, but is not limited to, MacPattern (Fuchs, 1991, Comput. Appl. Biosci. 7: 105-106), BLASTN and BLASTX (NCBI).

The present invention is further described by the following examples which should not be construed as limiting the scope of the invention.

### Examples

### Materials

Chemicals used as buffers and substrates were commercial products of at least reagent grade.

The antibiotics used herein as well as how they were prepared are listed in Table 1. The antibiotics were purchased from U.S. Pharmacopeia (Rockville, MD) or Sigma-Aldrich Corp. (St. Louis, MO).

**Table 1**

| **Antibiotic** | **Concentration of Stock** | **Solvent** |
|---|---|---|
| chloramphenicol | 5120 µg/ml | 95% EtOH |
| ciprofloxacin | 5120 µg/ml | water |
| cephalothin | 5120 µg/ml | water |
| vancomycin | 5120 µg/ml | water |
| erythromycin | 750 µg/ml or 5120 µg/ml | 50% EtOH or 95% EtOH |
| streptomycin | 5120 µg/ml | water |
| gentamicin | 5120 µg/ml | water |
| norfloxacin | 5120 µg/ml | 1/2 volume water, 0.1 mol/l NaOH dropwise to dissolve |
| trimethoprim | 5120 µg/ml | 0.05 mol/l HCl, 10% of final vol. |
| novobiocin | 5120 µg/ml | methanol |
| nalidixic acid | 5120 µg/ml | 1/2 volume water, then 1.0 M NaOH dropwise |
| gramicidin A | 5120 µg/ml | Methanol |
| rifampin | 5120 µg/ml | Methanol |

Mueller Hinton Broth (MHB) medium was composed per liter of 2 g of beef infusion, 17.5 g of acid digested casein, and 1.5 g of starch.

Tryptic soy agar medium was composed per liter of 15 g of tryptone peptone (pancreatic digestion of casein), 5 g of soytone peptone (papaic digest of soybean meal), 5 g of sodium chloride, and 15 g of agar.

LB plates consisted of per liter 10 g of tryptone, 5 g of yeast extract, and 5 g of sodium chloride.

*Bacillus subtilis* strain BGSC1A2 was obtained from the Bacillus Genetics Stock Center (Columbus, Ohio) and is a prototroph of *Bacillus subtilis* 168. The strain was maintained on LB agar.

### Example 1: Microbroth dilution assays

Microbroth dilution assays were performed as described in NCCLS M7-A5 (Vol. 20, No. 2). The assay was performed in a sterile 96 well plate, and the total volume per well was 100 µl. The inoculum was prepared to give approximately 10⁴ to 10⁵ colony forming units per well and the compounds were tested at concentrations from 0.0625 to 256 µg/ml in two-fold step dilution. The inoculum was prepared by culturing *Bacillus subtilis* BGSC1A2 overnight in MHB medium at 35°C with shaking at 200 rpm. The following day 0.5 ml of the overnight culture was used to inoculate MHB medium to obtain a culture in logarithmic phase growth as described in NCCLS M7-A5 (Vol. 20, No. 2) and M26-A (Vol. 19, No. 18). The actual colony forming units per well was confirmed by plating onto TSA or LB agar. Two wells were inoculated for a given concentration. The plates were incubated for 16 to 20 hours at 35°C The MIC was defined as that concentration of antibiotic resulting in no visible growth of the organism.

The results are shown in Table 2 below.

**Table 2**

| Antibiotic | MIC (µg/ml) |
|---|---|
| chloramphenicol | 2-4 |
| ciprofloxacin | 0.0625-0.125 |
| cephalothin | 0.0156-0.0625 |
| vancomycin | 0.125-0.25 |
| erythromycin | 0.125-0.25 |
| streptomycin | 4-8 |
| gentamicin | 0.125-0.25 |
| norfloxacin | 0.5 |
| trimethoprim | 0.25 |
| nalidixic acid | 4.0 |
| rifampin | 0.25-0.50 |
| novobiocin | 2.0 |
| gramicidin A | 8.0 |

### Example 2: Treatment of Bacillus subtilis cultures with sub-inhibitory concentrations of antibiotics

*Bacillus subtilis* strain BGSC1A2 was streaked onto a LB agar plate and incubated overnight at 37°C. The cultured plate was stored at room temperature for up to one week. Fifty ml of MHB medium in a 250 ml flask was inoculated with one colony from the plate. The culture was incubated overnight at 37°C and 200 rpm.

A 30.7 ml sample of the overnight culture was used to inoculate 3.6 liters of MHB medium pre-warmed at 37°C. After mixing the culture well, 600 ml aliquots were removed and placed into each of six 2.8 liter baffled shake flasks. A 1 ml sample of each flask was taken for optical density measurement at 600 nm, and the shake flasks were incubated at 37°C and 200 rpm. One ml samples were taken from each flask every 30 minutes until the OD₆₀₀ reached 0.2. At this point, which was designated time zero, 100 ml samples from each shake flask were taken, and 50 ml aliquots were placed in each of two 50 ml centrifuge tubes. In addition, 1.1 ml aliquots were taken from each flask with 1 ml used for an optical density measurement at 600 nm and 50 µl of each was placed in a microfuge tube at 4°C. The 50 ml samples were processed immediately by centrifugation at 4000 rpm at 4°C for 7 minutes, and after centrifugation the supernatant was decanted being careful not to disturb the pellet which was immediately placed at -80°C for total RNA isolation at a later time.

At time zero, different sub-inhibitory concentrations (0.1-1X MIC) of a given antibiotic were added to three of the shake flasks (Table 2) and the other three shake flasks were untreated. The shake flasks were incubated at 37°C and 200 rpm. At times 5, 15, 30 and 60 minutes after antibiotic addition, 100 ml and 1.1 ml samples were taken from each shake flask and processed as described above. After the 60 minute time point, 1.1 ml aliquots from each shake flask were taken every hour for four hours for optical density and cell viability measurements.

Cell viability was determined on all the samples by determining colony forming units per ml. Samples collected were diluted in MHB to obtain 10 to 500 colonies per plate as follows: samples collected at t=0 were diluted at 1:100,000; t=5 minutes, dilution was 1:150,000; t=15 minutes, dilution was 1:200,000; t=30 minutes, dilution was 1:250,000; t=1 hour, dilution was 1:300,000; t=1.5 hours, dilution was 1:400,000; t=2.5 hours, dilution was 1:800,000; and t=3.5 hours, dilution was 1:1,000,000. These dilutions factors can be changed as indicated by the response to the antibiotic being tested. For each diluted sample 20 µl and 40 µl aliquots were spread onto each of two pre-warmed LB agar plates, which were incubated overnight at 37°C. The number of colonies on each plate was counted to determine the colony forming units per ml of each sample.

The following antibiotics (Table 3) at the specified concentrations were tested.

**Table 3**

| **Antibiotic** | **Concentrations (µg/ml)** |
|---|---|
| chloramphenicol | 0.20, 1 & 1.6 |
| ciprofloxacin | 0.0125, 0.0625 & 0.125 |
| cephalothin | 0.00156, 0.0078 & 0.0156 |
| vancomycin | 0.0125, 0.0625 & 0.125 |
| erythromycin | 0.0125, 0.03125 & 0.0625 |
| streptomycin | 0.80, 4 & 8 |
| gentamicin | 0.0125, 0.03125 & 0.05 |
| norfloxacin | 0.05, 0.125 & 0.25 |
| trimethoprim | 0.025, 0.0625 & 0.080 |
| rifampin | 0.0025, 0.005, & 0.0125 |

### Example 3: Isolation of Total RNA from Shake Flasks Samples

The cell pellets from Example 2 were thawed on ice, and RNA was prepared using the FastRNA^{™} Blue Kit (BIO101) (QBIOgene, Carlsbad, CA), according to the manufacturer's instructions, with minor modifications. Two hundred microliters of cell suspension was added to each FastPrep^{™} blue tube containing the lysing matrix as well as the other required reagents as listed in the protocol. The total number of tubes per frozen sample was adjusted depending on the volume but was typically three to four FastPrep^{™} blue tubes per pellet. After addition of the cell suspension to the FastPrep^{™} blue tubes, the tubes were processed in the FastPrep^{™} instrument at a speed rating of 6, one time for 45 seconds, and placed on ice to cool prior to opening. The samples were centrifuged as per the protocol, and the top phase was collected and combined with replicate samples in a Falcon 2059 polypropylene tube. Appropriate volumes of reagents, as listed in the protocol, were added to the pooled samples, which were processed as described in the manufacturer's protocols. The final RNA pellet was resuspended in 50 µl of SAFE buffer (provided in the FastRNA kit), and each RNA sample was analyzed on agarose gels to assess quality. In addition, quantity/quality for each RNA preparation was determined by measuring the optical density at 260 and 280 nm and calculating the ratio of ODZ₂₆₀/OD₂₈₀. Samples were frozen at -80°C.

### Example 4: Preparation of DNA Microarrays

A complete set of *Bacillus subtilis* ORF-specific PCR primers was purchased from Eurogentec (Seraing, Belgium) and used to amplify the protein coding ORFs from *Bacillus subtilis* BGSC1A2 genomic DNA. Chromosomal DNA was prepared using the method described by Pitcher et al., 1989, Lett. Appl. Microbiol. 8: 151-156. The following PCR components were combined in 96-well plates: 25 pmol of each primer, 1 x *Taq* polymerase buffer (PE Applied Biosystems, Foster City, CA), 0.25 mM dATP, 0.25 mM dCTP, 0.25 mM dGTP, 0.25 mM TTP, 2 mM MgCl₂, 0.1 µg of *Bacillus subtilis* genomic DNA, and 1.75 U of *Taq* polymerase (PE Applied Biosystems). An MJ Research PTC-225 thermocycler (MJ Research, Inc., Waltham, MA) was programmed to incubate the reactions for 36 cycles each at 95°C for 30 seconds), 56°C for 45 seconds, and 72°C for 3 minutes 30 seconds.

An aliquot of each completed reaction was analyzed by agarose gel electrophoresis to ensure that a PCR product of the correct size and adequate yield was obtained. The success rate for a single pass amplification of the 4107 ORFs was about 92%. Failed reactions, most often occurring for genes with long open reading frames (ca. 3 to 15 kb), were repeated using Expand™ polymerase mixture (Roche Molecular Biochemicals, Indianapolis, IN) and the reaction products were verified by agarose gel electrophoresis. Collectively, the PCR product pools generated in two rounds of amplifications represented approximately 95% of the *Bacillus subtilis* genome.

The amplified *Bacillus subtilis* ORFs were precipitated with isopropanol, resuspended in 15 µl of 3X SSC, and 5 µl aliquots were stored at -20°C in 384-well microplates (Eisen and Brown, 1999, Methods Enzymol. 303: 179-205). From these plates, the ORFs were spotted onto poly-L-lysine coated glass microscope slides using the equipment and methods that are described on the web site of P.O. Brown of Stanford University (http://cmgm.stanford.edu/pbrown/protocols).

Arrays were also made as described above with a complete set of oligonucleotides (65mers to each of the 4100 open reading frames of *Bacillus subtilis*) purchased from Compugen (Jamesburg, New Jersey). The oligonucleotides were provided dried and were resuspended prior to printing in 3X SSC at a final concentration of 10 and 20 µM followed by printing onto poly-L-lysine coated glass microscope slides as described above.

### Example 5: Probe Preparation and Hybridization

Fluorescent probes were prepared by reverse transcription of 25 µg of total RNA from *Bacillus subtilis* to incorporate aminoallyl-dUTP into first strand cDNA. RNA was mixed with 1 µg random primer (9 mers) (New England Biolab, Beverly, MA), and incubated at 70°C for 10 minutes before chilled on ice for 10 minutes The first strand cDNA synthesis was completed by adding 1X SuperScript buffer (Invitrogen, Carlsbad, CA), 500 µM each of dATP, dCTP, dGTP, 300 µM dTTP, and 200 µM 5-(3-aminoallyl)-, 2'-deoxyuridine 5' triphosphate to the RNA/primer mixture in the presence of 10 mM DTT and 380 U of Superscript II RNase H⁻ reverse transcriptase (Invitrogen, Carlsbad, CA). The mixture was incubated at 42°C for 2 hours. The RNA template was then hydrolyzed by bringing cDNA synthesis reactions to a final concentration of 200 mM NaOH and 100 mM EDTA, and incubated at 65°C for 15 minutes. The hydrolysis reaction was then neutralized by addition of Tris (pH 7.4) to a final concentration of 333 mM. The Tris was then removed from the reaction by passing through Microcon 30 concentrator (Millipore, Beford, MA) three times with 450 µl of water to prevent the monofunctional NHS-ester Cye dyes from coupling to free amine groups in solution. The amino-allyl labeled cDNA was dried and stored at -20°C before further processing.

The cDNA products were subsequently labeled by direct coupling to either Cy3 or Cy5 monofunctional reactive dyes (Amersham Pharmacia Biotech, Arlington Heights, IL). Cy3 and Cy5 dyes were resuspended in 72 µl of DMSO, dispensed as 4.5 µl aliquots, and dried down in a speed vacuum, and stored at 4°C in a vacuum dessicator. The cDNA pellet was resuspended in 9 µl of 50 mM sodium bicarbonate pH 9 buffer, before adding it to the tube containing an aliquot of the Cy3 or Cy5 dyes. Typically, the control cDNAs were mixed with Cy3 and cDNAs isolated from treated samples were mixed with Cy5. The reaction was incubated at room temperature in the dark for 1 hour. The reaction was then quenched to prevent cross-coupling by adding 1 M hydroxylamine and incubating at room temperature in the dark for 15 minutes. The Cy3 and Cy5 reactions were combined for each corresponding samples (*i.e.*, mixing control and treatment samples), and the unincorporated or quenched cye dyes were removed using the QIAquick PCR purification kit (Qiagen, Valencia, CA). The purified probes were dried under vacuum in a SpeedVac (Savant Instruments, Inc., Holbrook, NY), resuspended in 15.5 µl of water and combined with the following: 3.6 µl of 20X SSC, 2.5 µl of 250 mM HEPES (pH 7.0), 1.8 µl of poly-dA (500 µg/ml; Amersham Pharmacia Biotech), and 0.54 µl of 10% SDS. Before hybridization, the solution was filtered with a 0.22 µm Ultrafree-MC microcentrifuge filter (Millipore, Beford, MA), boiled for 2 minutes and cooled to room temperature. The probe was then applied to the microarray under a coverglass, placed in a humidified chamber, and incubated at 62-65°C overnight. Before scanning, the arrays were washed consecutively in 1xSSC with 0.03% SDS, 0.2X SSC, and 0.05X SSC and centrifuged for 2 minutes at 500 rpm to remove excess liquid. Lastly, the slides were imaged using a GenePix 4000B scanner and the image was acquired with GenePix Pro 3.0 software (Axon Instruments, Union City, CA).

### Example 6: Data Normalization

Using the Genepix Pro software, the median of the local background was subtracted from each feature for both the 532 nm and 635 nm wavelengths. The median signal intensity for each feature at both wavelengths was then imported into GeneSpring 4.2 where the following normalization procedures were performed. Each gene's measured intensity was divided by its control channel value in each sample. When the control channel value was below 10.0 the data point was considered bad. Intensity-dependent normalization was also applied, where the ratio was reduced to the residual of the Lowess fit of the intensity versus ratio curve. The 50th percentile of all measurements was used as a positive control for each sample. Each measurement for each gene was divided by this synthetic positive control, assuming that this was at least 0.01. Only genes marked as marginal or present were used. The bottom tenth percentile was used as a test for correct background subtraction, which was never less than the negative of the synthetic positive control. Lastly, normalized values below 0 were set to 0.

### Example 7: Data Analysis and Mining

The normalized data representing a culture treated with one drug concentration at a single time point was exported from GeneSpring (Silicon Genetics, Redwood, CA) and evaluated with the SAM software (Significance Analysis of Microarrays; Tusher et al., 2001, Proc. Nat. Acad. Sci. USA 98:5116-5121). All genes considered up or down regulated by SAM as one-class response, with a false discovery rate of less then one, were imported back into GeneSpring where the expression profile was analyzed. The significant genes were first analyzed by the filtering and statistical analysis tool of GeneSpring to screen for genes that showed at least 1.5-fold change in expression. A signature for each drug was obtained by combining the expression data of all genes called up or down regulated at any time point (except for time 0) and concentrations of drug treatment. Patterns of gene expression can be further characterized with standard clustering techniques, such as Hierarchical and k-means, or by generating self organizing maps.

In addition, the Class Predictor tool in the GeneSpring software package can be used to compile a list of predictor genes for each class of antibiotics. The Class Predictor is a fully automated tool within GeneSpring that allows the identification of key predictor genes that differentiate between biological states (for example, treatment with different antibiotics). The key predictive genes can then be subsequently used to predict the mode-of-action of a novel antibiotic. Alternatively, the Prediction Analysis for Microarray (PAM) program which performs sample classification from gene expression data via a nearest shrunken centroid method can be used for this same purpose (Tibshirani et al., 2002, Proc. Nat. Acad. Sci. USA 99: 6567-6572). PAM provides a list of significant genes whose expression characterizes each class which can be used to predict the classification of a novel compound. The small subset of predictive genes can be used to make an array containing the minimum subset of genes needed to predict if a new compound falls into a pre-existing class (mode-of-action) of antibiotics.

### Example 8: Results of treatment with chloramphenicol

*Bacillus subtilis* cultures were treated with sub-inhibitory concentrations of chloramphenicol as described in Example 2. The chloramphenicol concentrations used were 0.20, 1 and 1.6 µg/ml, which corresponded to 0.05, 0.25 and 0.4 times the minimum inhibitory concentration. The growth curve generated by plotting the log of the optical density measurements at 600 nm against time in minutes after antibiotics were added showed the cultures treated with 1 or 1.6 µg/ml showed a decrease in growth in comparison to the three untreated controls and the culture treated with 0.20 µg/ml. The colony forming units of each sample were also determined as described in Example 2 and plotted versus time in minutes after antibiotic addition. As observed in the growth curve, the colony forming units were significantly affected in the cultures treated with 1 or 1.6 µg/ml chloramphenicol compared to the untreated control or the culture treated with 0.2 µg/ml.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was done. The colony forming units and growth curves were similar to those observed in the first shake flask experiments.

### Example 9: Results of treatment with ciprofloxacin

*Bacillus subtilis* cultures were treated with sub-inhibitory concentrations of ciprofloxacin as described in Example 2. The ciprofloxacin concentrations used were 0.0125, 0.0625 and 0.125 µg/ml, which corresponded to 0.1, 0.50 and 1.0 times the minimum inhibitory concentration, respectively. The growth curve generated by plotting the log of the optical density measurements at 600 nm against time in minutes after antibiotics were added showed the ciprofloxacin treatment had very little effect, if any, on growth rate as measured by optical density. In contrast, treatment with ciprofloxacin led to a decrease in colony forming units or viability, and this effect was does dependent.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was performed and similar effects on growth as measured by optical density and viability as measured by colony forming units were observed.

### Example 10: Results of treatment with cephalothin

*Bacillus subtilis* cultures were treated with sub-inhibitory concentrations of cephalothin as described in Example 2. The cephalothin concentrations used were 0.00156, 0.0078 and 0.0156 µg/ml, which corresponded to 0.1, 0.50 and 1.0 times the minimum inhibitory concentration, respectively. The growth curve generated by plotting the log of the optical density measurements at 600 nm against time in minutes after antibiotics were added showed the cephalothin treatment at 0.0156 and 0.0078 µg/ ml led to a large and moderate decrease, respectively, in growth rate as measured by optical density while the 0.00156 treatment had very little effect, if any, on growth. Similarly, treatment with cephalothin led to a decrease in colony forming units or viability, and this effect was dose dependent as observed for the growth rate.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was performed. The growth curves and colony forming units were plotted and were very similar to those from the first experiment.

### Example 11: Results of treatment with norfloxacin

*Bacillus subtilis* cultures were treated with sub-inhibitory concentrations of norfloxacin as described in Example 2. The norfloxacin concentrations used were 0.05, 0.125 and 0.25 µg/ml, which corresponded to 0.1, 0.25 and 0.5 times the minimum inhibitory concentration, respectively. The growth curve generated by plotting the log of the optical density measurements at 600 nm against time in minutes after antibiotics were added showed the norfloxacin treatment had very little effect, if any, on growth rate as measured by optical density; this is similar to the effect of ciprofloxacin which has the same mode of action as ciprofloxacin. In contrast, treatment with norfloxacin led to a decrease in colony forming units or viability, and this effect was dose dependent as expected.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was done. The growth curves and colony forming units were plotted and were very similar to those from the first experiment.

### Example 12: Results of treatment with vancomycin

*Bacillus subtilis* cultures were treated with sub-inhibitory concentrations of vancomycin as described in Example 2. The vancomycin concentrations used were 0.0125, 0.0625 and 0.125 µg/ml, which corresponded to 0.1, 0.25 and 0.5 times the minimum inhibitory concentration, respectively. The growth curve generated by plotting the log of the optical density measurements at 600 nm against time in minutes after antibiotics were added showed the vancomycin treatment had very little effect, if any, on growth rate as measured by optical density. In addition, the concentrations of vancomycin used also had no effect on viability over the time period sampled.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was done. The growth curves and colony forming units were plotted and look very similar to those from the first experiment.

### Example 13: Results of treatment with streptomycin

*Bacillus subtilis* cultures were treated with sub-inhibitory concentrations of streptomycin as described in Example 2. The streptomycin concentrations used were 0.8, 4.0, and 8.0 µg/ml, which corresponded to 0.1, 0.50 and 1.0 times the minimum inhibitory concentration, respectively. The growth curve generated by plotting the log of the optical density measurements at 600 nm against time in minutes after antibiotics were added showed the streptomycin treatment at 8.0 and 4.0 µg/ml showed a large and moderate decrease in growth rate, respectively, while the 0.8 µg/ml treated culture had a growth rate similar to the untreated cultures. Similarly, treatment with streptomycin led to a decrease in colony forming units or viability, and this effect was dose dependent.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was done. The growth curves and colony forming units were plotted and look very similar to those from the first experiment

### Example 14: Results of treatment with gentamicin

*Bacillus subtilis* cultures were treated with sub-inhibitory concentrations of gentamicin as described in Example 2. The gentamicin concentrations used were 0.0125, 0.03125 and 0.05 µg/ml, which corresponded to 0.1, 0.25 and 0.4 times the minimum inhibitory concentration, respectively. The growth curve generated by plotting the log of the optical density measurements at 600 nm against time in minutes after antibiotics were added showed the gentamicin treatment had very little effect if any on growth rate as measured by optical density. In contrast, treatment with gentamicin led to a decrease in colony forming units or viability, and this effect was dose dependent.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was done. The growth curves and colony forming units were plotted and were very similar to those from the first experiment

### Example 15: Results of treatment with erythromycin

*Bacillus subtilis* cultures were treated with sub-inhibitory concentrations of erythromycin as described in Example 2. The erythromycin concentrations used were 0.0125, 0.03125 and 0.0625µg/ml, which corresponded to 0.1, 0.25 and 0.5 times the minimum inhibitory concentration, respectively. The growth curve generated by plotting the log of the optical density measurements at 600 nm against time in minutes after antibiotics were added showed the erythromycin treatment caused a decrease in growth rate that was dose dependent. Similarly, treatment with erythromycin led to a decrease in colony forming units or viability, and this effect was dose dependent.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was done. The growth curves and colony forming units were plotted and look very similar to those from the first experiment

### Example 16: Results of treatment with munumbicin B

To determine the mode of action of munumbicin B (Uvidelio et al., 2002, Microbiology 148: 2675-2685), *Bacillus subtilis* cultures were treated with sub-inhibitory concentrations of munumbicin B as described in Example 2. The munumbicin B concentrations used were 0.0125, 0.03125 and 0.0625 µg/ml, which corresponded to 0.1, 0.25 and 0.5 times the minimum inhibitory concentration, respectively. The growth curve generated by plotting the log of the optical density measurements at 600 nm against time in minutes after antibiotics were added showed the munumbicin B treatment at 0.03125 and 0.0625 µg/ml caused a decrease in growth rate that was dose dependent while the culture treated with 0.0125 µg/ml grew similar to the untreated cultures. Similarly, treatment with munumbicin B led to a decrease in colony forming units or viability, and this effect was dose dependent.

Total RNA was extracted as described in Example 3 from the samples collected at 0, 15, 30 and 60 minutes after antibiotic addition and probes were prepared and hybridized to the DNA microarrays as described in Example 5.

### Example 17: Results of treatment with trimethoprim

*Bacillus subtilis* cultures were treated with sub-inhibitory concentrations of trimethoprim as described in Example 2. The trimethoprim concentrations used were 0.025, 0.0625 and 0.08 µg/ml, which corresponded to 0.1, 0.25 and 0.4 times the minimum inhibitory concentration, respectively. The growth curve generated by plotting the log of the optical density measurements at 600 nm against time in minutes after antibiotics were added showed the trimethoprim treatment did not have an effect on growth rate. Similarly, treatment with trimethoprim did not lead to a change in colony forming units or viability.

Total RNA was extracted as described in Example 3 from the samples collected at 0, 15, 30 and 60 minutes after antibiotic addition and probes were prepared and hybridized to the DNA microarrays as described in Example 5.

### Example 18: Results of treatment with rifampin

*Bacillus subtilis* cultures were treated with sub-inhibitory concentrations of rifampin as described in Example 2, respectively. The rifampin concentrations used were 0.0025, 0.005 and 0.0125 µg/ml, which corresponded to 0.01, 0.02 and 0.05 times the MIC, respectively. The growth curve generated by plotting the log of the optical density measurements at 600 nm against time in minutes after antibiotics were added showed the rifampin treatment at 0.01 and 0.02 X MIC had very little effect if any on growth rate as measured by optical density while the treatment at 0.05X caused some growth inhibition. Similarly, treatment with rifampin at 0.01 or 0.02 X MIC had no effect on colony forming units while treatment at 0.05X led to a decrease in colony forming units or viability.

Total RNA was extracted as described in Example 3 from the samples collected at 0, 15, 30 and 60 minutes after antibiotic addition and probes were prepared and hybridized to the DNA microarrays as described in Example 5.

A duplicate experiment to the one described above was done. The growth curves and colony forming units were plotted and were very similar to those from the first experiment.

### Example 19: List of genes that are significantly differentially expressed due to antibiotic treatment

Samples from the cultures treated with the various antibiotics as described in Examples 8 through 18 were processed and analyzed as described in Examples 2 through 7. The list of genes, which were significantly overexpressed or underexpressed in comparison to an untreated culture, were determined for each antibiotic and contained data for several time points and concentrations as described in the above examples. These lists were further analyzed as described in Examples 20 to 21.

### Example 20: Comparisons of results for compounds from related classes

The list of genes described in Example 19 were analyzed to make lists of genes that were common across all antibiotics and genes that were common to a class of antibiotics based on mode of action. In addition, the lists were analyzed to determine genes that were uniquely upregulated or downregulated due to treatment with a particular class of antibiotics. These lists were used to identify genes that can be used as reporters for a particular mode of action or as potential targets for antimicrobial drug development. The lists can also be used to identify a subset of the *Bacillus subtilis* genes that could be used to generate a DNA microarray that could be used in a high throughput fashion to determine the mode of action of a new antimicrobial compound.

Tables 4 and 5 list the genes that were upregulated and downregulated, respectively, due to treatment with either ciprofloxacin or norfloxacin.

**Table 4**

| **Gene** | **Accession number** | **Description** |
|---|---|---|
| atpb | bg10815 | ATP synthase (subunit a) |
| clpp | bg19016 | ATP-dependent Clp protease proteolytic subunit (class III heat-shock protein) |
| infa | bg11043 | initiation factor IF-1 |
| ldh | bg19003 | L-lactate dehydrogenase |
| lica | bg11349 | PTS lichenan-specific enzyme IIA component |
| lica | bg11349 | PTS lichenan-specific enzyme IIA component |
| lica | bg11349 | PTS lichenan-specific enzyme IIA component |
| lica | bg11349 | PTS lichenan-specific enzyme IIA component |
| lica | bg11349 | PTS lichenan-specific enzyme IIA component |
| oppf | bg10775 | "oligopeptide ABC transporter (ATP-binding protein) (initiation of sporulation, competence development)" |
| pdp | bg10985 | pyrimidine-nucleoside phosphorylase |
| reca | bg10721 | multifunctional protein involved in homologous recombination and DNA repair (LexA-autocleavage) |
| rpla | bg10164 | ribosomal protein L1 (BL1) |
| rpld | bg11219 | ribosomal protein L4 |
| rple | bg10760 | ribosomal protein L5 (BL6) |
| rplk | bg10163 | ribosomal protein L11 (BL11) |
| rplr | bg11409 | ribosomal protein L18 |
| rplv | bg11078 | ribosomal protein L22 (BL17) |
| rplw | bg11221 | ribosomal protein L23 |
| rpme | bg10417 | ribosomal protein L31 |
| rpsc | bg19005 | ribosomal protein S3 (BS3) |
| rpsj | bg19008 | ribosomal protein S10 (BS13) |
| rpso | bg19010 | ribosomal protein S15 (BS1 8) |
| rpsq | bg10757 | ribosomal protein S17 (BS16) |
| soda | bg11676 | superoxide dismutase |
| uvra | bg12697 | excinuclease ABC (subunit A) |
| uvrb | bg10502 | excinuclease ABC (subunit B) |
| uvrb | bg10502 | excinuclease ABC (subunit B) |
| uvrb | bg10502 | excinuclease ABC (subunit B) |
| ybxf | bg11365 | unknown; similar to ribosomal protein L7AE family |
| ydas | bg12066 | unknown |
| ydas | bg12066 | unknown |
| ydip | bg12788 | unknown; similar to DNA-methyltransferase (cytosine-specific) |
| ydip | bg12788 | unknown; similar to DNA-methyltransferase (cytosine-specific) |
| yerq | bg12843 | unknown; similar to unknown proteins |
| yhdh | bg13014 | unknown; similar to sodium-dependent transporter |
| yhdh | bg13014 | unknown; similar to sodium-dependent transporter |
| ylbn | bg13366 | unknown; similar to unknown proteins |
| ylxq | bg10267 | unknown; similar to ribosomal protein L7AE family |
| yosn | bg13723 | unknown; similar to ribonucleoside-diphosphate reductase (alpha subunit) |
| ypfd | bg11005 | unknown; similar to ribosomal protein S1 homolog |
| ypfd | bg11005 | unknown; similar to ribosomal protein S1 homolog |
| ypfd | bg11005 | unknown; similar to ribosomal protein S1 homolog |
| yrbf | bg13785 | unknown; similar to unknown proteins |
| yubb | bg13951 | unknown; similar to bacitracin resistance protein (undecaprenol kinase) |
| yvce | bg11023 | unknown; similar to cell wall-binding protein |
| yvce | bg11023 | unknown; similar to cell wall-binding protein |
| yvdf | bg12415 | "unknown; similar to glucan 1,4-alpha-maltohydrolase" |
| yvdf | bg12415 | "unknown; similar to glucan 1,4-alpha-maltohydrolase" |
| yvdf | bg12415 | "unknown; similar to glucan 1,4-alpha-maltohydrolase" |
| yvdf | bg12415 | "unknown; similar to glucan 1,4-alpha-maltohydrolase" |
| ywpb | bg12496 | unknown; similar to hydroxymyristoyl-(acyl carrier protein) dehydratase |
| yyaa | bg10057 | unknown; similar to DNA-binding protein Spo0J-like |

**Table 5**

| **Gene** | **Accession number** | **Description** |
|---|---|---|
| rpsr | bg10047 | ribosomal protein S18 |
| rpmh | bg10064 | ribosomal protein L34 |
| abrb | bg10100 | transcriptional pleiotropic regulator of transition state genes |
| glpk | bg10187 | glycerol kinase |
| ptsh | bg10200 | histidine-containing phosphocarrier protein of the PTS (HPr protein) |
| ptsi | bg10201 | PTS enzyme I |
| maf | bg10324 | septum formation |
| rpse | bg10442 | ribosomal protein S5 |
| rpmd | bg10443 | ribosomal protein L30 (BL27) |
| adk | bg10446 | adenylate kinase |
| map | bg10447 | methionine aminopeptidase |
| glpq | bg10646 | glycerophosphoryl diester phosphodiesterase |
| mota | bg10688 | motility protein (flagellar motor rotation) |
| motb | bg10689 | motility protein (flagellar motor rotation) |
| pure | bg10700 | phosphoribosylaminoimidazole carboxylase I |
| purm | bg10708 | phosphoribosylaminoimidazole synthetase |
| purn | bg10709 | phosphoribosylglycinamide formyl transferase |
| rpll | bg10726 | ribosomal protein L12 (BL9) |
| rpsm | bg10730 | ribosomal protein S13 |
| rpsk | bg10731 | ribosomal protein S11 (BS11) |
| rpmc | bg10756 | ribosomal protein L29 |
| rpsq | bg10757 | ribosomal protein S17 (BS16) |
| rpln | bg10758 | ribosomal protein L14 |
| rplx | bg10759 | ribosomal protein L24 (BL23) (histone-like protein HPB12) |
| rple | bg10760 | ribosomal protein L5 (BL6) |
| rpsn | bg10761 | ribosomal protein S14 |
| rpsh | bg10762 | ribosomal protein S8 (BS8) |
| rpmb | bg10777 | ribosomal protein L28 |
| gapa | bg10827 | glyceraldehyde-3-phosphate dehydrogenase |
| rpsp | bg10831 | ribosomal protein S16 (BS17) |
| ask | bg10915 | aspartokinase II attenuator |
| flis | bg10922 | flagellar protein |
| glya | bg10944 | serine hydroxymethyltransferase |
| rpmj | bg11042 | ribosomal protein L36 (ribosomal protein B) |
| infa | bg11043 | initiation factor IF-1 |
| rplv | bg11078 | ribosomal protein L22 (BL17) |
| pbux | bg11080 | xanthine permease |
| rplb | bg11217 | ribosomal protein L2 (BL2) |
| rpld | bg11219 | ribosomal protein L4 |
| rplj | bg11220 | ribosomal protein L10 (BL5) |
| rplw | bg11221 | ribosomal protein L23 |
| rplf | bg11408 | ribosomal protein L6 (BL8) |
| rplr | bg11409 | ribosomal protein L18 |
| acpa | bg11536 | acyl carrier protein |
| rpst | bg11643 | ribosomal protein S20 (BS20) |
| yqfp | bg11662 | unknown; similar to penicillin tolerance |
| yqhl | bg11700 | unknown; similar to unknown proteins |
| frua | bg11938 | PTS fructose-specific enzyme IIABC component |
| infc | bg11944 | initiation factor IF-3 |
| rplt | bg11971 | ribosomal protein L20 |
| rpmi | bg11972 | ribosomal protein L35 |
| dctp | bg12075 | C4-dicarboxylate transport protein |
| ydbl | bg12079 | unknown |
| ysba | bg12311 | unknown |
| ysbb | bg12312 | unknown; similar to unknown proteins |
| yxjj | bg12540 | unknown |
| frur | bg12589 | transcriptional repressor of the fructose operon |
| Imra | bg12612 | transcriptional repressor of the lincomycin operon |
| pfka | bg12644 | 6-phosphofructokinase |
| rpmf | bg12668 | ribosomal protein L32 |
| pbug | bg12811 | hypoxanthine/guanine permease |
| yetj | bg12866 | unknown; similar to unknown proteins |
| yetk | bg12867 | unknown; similar to unknown proteins from B. subtilis |
| nagp | bg12941 | putative PTS N-acetylglucosamine-specific enzyme IICB component |
| yfms | bg12970 | unknown; similar to methyl-accepting chemotaxis protein |
| yfmt | bg12971 | unknown; similar to benzaldehyde dehydrogenase |
| yhdo | bg13021 | unknown; similar to 1-acylglycerol-3-phosphate O-acyltransferase |
| hemat | bg13066 | haem-based aerotactic transducer |
| yjbj | bg13139 | unknown; similar to lytic transglycosylase |
| fabi | bg13152 | enoyl-acyl carrier protein reductase |
| manp | bg13176 | putative PTS mannose-specific enzyme IIBCA component |
| yjfb | bg13186 | unknown |
| ykba | bg13227 | unknown; similar to amino acid permease |
| ykok | bg13256 | unknown; similar to Mg2+ transporter |
| ykom | bg13258 | unknown; similar to transcriptional regulator (MarR family) |
| ykrl | bg13274 | unknown; similar to heat-shock protein |
| ylqb | bg13401 | unknown |
| yoeb | bg13549 | unknown |
| yola | bg13580 | unknown |
| yolb | bg13581 | unknown; similar to phage-related protein |
| ypze | bg13767 | unknown |
| ytip | bg13864 | unknown; similar to unknown proteins |
| ytlq | bg13879 | unknown; similar to unknown proteins |
| yxzc | bg14167 | unknown |
| rpsc | bg19005 | ribosomal protein S3 (BS3) |
| rpsl | bg19009 | ribosomal protein S12 (BS12) |
| rpss | bg19011 | ribosomal protein S19 (BS19) |

Tables 6 and 7 below list the genes that were uniquely up or downregulated, respectively, due to treatment with either ciprofloxacin or norfloxacin. The genes in Table 6 were upregulated due to treatment with ciprofloxacin or norfloxacin but not due to treatment with cephalothin, vancomycin or trimethoprim. The genes in Table 7 were downregulated due to treatment with ciprofloxacin and norfloxacin but not due to any of the other antibiotics.

**Table 6**

| **Gene** | **Accession number** | **Description** |
|---|---|---|
| clpp | bg19016 | ATP-dependent Clp protease proteolytic subunit (class III heat-shock protein) |
| lica | bg11349 | PTS lichenan-specific enzyme IIA component |
| oppf | bg10775 | "oligopeptide ABC transporter (ATP-binding protein) (initiation of sporulation, competence development)" |
| pdp | bg10985 | pyrimidine-nucleoside phosphorylase |
| reca | bg10721 | multifunctional protein involved in homologous recombination and DNA repair (LexA-autocleavage) |
| rpso | bg19010 | ribosomal protein S15 (BS18) |
| uvra | bg12697 | excinuclease ABC (subunit A) |
| uvrb | bg10502 | excinuclease ABC (subunit B) |
| ydip | bg12788 | unknown; similar to DNA-methyltransferase (cytosine-specific) |
| yosn | bg13723 | unknown; similar to ribonucleoside-diphosphate reductase (alpha subunit) |
| ypfd | bg11005 | unknown; similar to ribosomal protein S1 homolog |
| yrbf | bg13785 | unknown; similar to unknown proteins |
| yubb | bg13951 | unknown; similar to bacitracin resistance protein (undecaprenol kinase) |
| yvce | bg11023 | unknown; similar to cell wall-binding protein |
| yvdf | bg12415 | "unknown; similar to glucan 1,4-alpha-maltohydrolase" |
| ywpb | bg12496 | unknown; similar to hydroxymyristoyl-(acyl carrier protein) dehydratase |
| yyaa | bg10057 | unknown; similar to DNA-binding protein Spo0J-like |

**Table 7**

| **Gene** | **Accession number** | **Description** |
|---|---|---|
| abrb | bg10100 | transcriptional pleiotropic regulator of transition state genes |
| flis | bg10922 | flagellar protein |
| gapa | bg10827 | glyceraldehyde-3-phosphate dehydrogenase |
| maf | bg10324 | septum formation |
| nagp | bg12941 | putative PTS N-acetylglucosamine-specific enzyme IICB component |
| pfka | bg12644 | 6-phosphofructokinase |
| ptsi | bg10201 | PTS enzyme I |
| pure | bg10700 | phosphoribosylaminoimidazole carboxylase I |
| purm | bg10708 | phosphoribosylaminoimidazole synthetase |
| rplv | bg11078 | ribosomal protein L22 (BL17) |
| rpsl | bg19009 | ribosomal protein S12 (BS12) |
| yetk | bg12867 | unknown; similar to unknown proteins from B. subtilis |
| yhdo | bg13021 | unknown; similar to 1-acylglycerol-3-phosphate O-acyltransferase |
| ykba | bg13227 | unknown; similar to amino acid permease |
| ykok | bg13256 | unknown; similar to Mg2+ transporter |
| yqhl | bg11700 | unknown; similar to unknown proteins |
| yxzc | bg14167 | unknown |

Table 8 and 9 below lists the common genes that were upregulated and downregulated, respectively, due to treatment with the cell wall inhibitor cephalothin or vancomycin.

**Table 8**

| **Gene** | **Accession number** | **Description** |
|---|---|---|
| dlte | bg10547 | involved in lipoteichoic acid biosynthesis |
| rpld | bg11219 | ribosomal protein L4 |
| rplj | bg11220 | ribosomal protein L10 (BL5) |
| rplw | bg11221 | ribosomal protein L23 |
| rpmi | bg11972 | ribosomal protein L35 |
| rpsf | bg10049 | ribosomal protein S6 (BS9) |
| rpsj | bg19008 | ribosomal protein S10 (BS13) |
| ydao | bg12062 | unknown; similar to unknown proteins |
| ywoa | bg12488 | unknown; similar to bacteriocin transport permease |

**Table 9**

| **Gene** | **Accession number** | **Description** |
|---|---|---|
| ahpc | bg11385 | alkyl hydroperoxide reductase (small subunit) |
| ald | bg10468 | L-alanine dehydrogenase |
| bofc | bg11917 | forespore regulator of the sigma-K checkpoint |
| citb | bg10478 | aconitate hydratase |
| citz | bg10855 | citrate synthase II (major) |
| ctaa | bg10213 | cytochrome caa3 oxidase (required for biosynthesis) |
| ctab | bg10214 | cytochrome caa3 oxidase (assembly factor) |
| cyda | bg11925 | cytochrome bd ubiquinol oxidase (subunit I) |
| feub | bg10836 | iron-uptake system (integral membrane protein) |
| glya | bg10944 | serine hydroxymethyltransferase |
| Ictp | bg12001 | L-lactate permease |
| malp | bg11848 | PTS maltose-specific enzyme IICB component |
| mmgd | bg11322 | citrate synthase III |
| mota | bg10688 | motility protein (flagellar motor rotation) |
| motb | bg10689 | motility protein (flagellar motor rotation) |
| mtla | bg11215 | PTS mannitol-specific enzyme IICBA component |
| ndk | bg10282 | nucleoside diphosphate kinase |
| nfra | bg10589 | FMN-containing NADPH-linked nitro/flavin reductase |
| phrf | bg11960 | phosphatase (RapF) regulator |
| purn | bg10709 | phosphoribosylglycinamide formyltransferase |
| qcrb | bg11326 | menaquinol:cytochrome c oxidoreductase (cytochrome b subunit) |
| rplc | bg11218 | ribosomal protein L3 (BL3) |
| rple | bg10760 | ribosomal protein L5 (BL6) |
| rpll | bg10726 | ribosomal protein L12 (BL9) |
| rpmb | bg10777 | ribosomal protein L28 |
| rpmc | bg10756 | ribosomal protein L29 |
| rpmd | bg10443 | ribosomal protein L30 (BL27) |
| rpmga | bg14180 | possible ribosomal protein L33 |
| rpsh | bg10762 | ribosomal protein S8 (BS8) |
| rpsk | bg10731 | ribosomal protein S11 (BS11) |
| soda | bg11676 | superoxide dismutase |
| srfac | bg10170 | surfactin synthetase / competence |
| tatcy | bg12207 | component of the twin-arginine translocation pathway |
| tkt | bg11247 | transketolase |
| ycsa | bg11222 | unknown; similar to 3-isopropylmalate dehydrogenase |
| ydas | bg12066 | unknown |
| ydil | bg12209 | unknown; similar to unknown proteins |
| yetg | bg12863 | unknown; similar to unknown proteins |
| yhag | bg12983 | unknown |
| yjbj | bg13139 | unknown; similar to lytic transglycosylase |
| yjch | bg13161 | unknown; similar to unknown proteins |
| ykoa | bg13247 | unknown |
| ykom | bg13258 | unknown; similar to transcriptional regulator (MarR family) |
| yloh | bg13387 | unknown; similar to unknown proteins |
| ylqb | bg13401 | unknown |
| yolf | bg13584 | unknown; similar to unknown proteins |
| ypaa | bg11428 | unknown |
| ypib | bg11497 | unknown; similar to unknown proteins |
| yqdb | bg11512 | unknown |
| yqgw | bg11690 | unknown |
| yrbf | bg13785 | unknown; similar to unknown proteins |
| ysba | bg12311 | unknown |
| yugm | bg12367 | unknown |
| yugn | bg12368 | unknown; similar to unknown proteins |
| yvfh | bg11875 | unknown; similar to L-lactate permease |
| ywdd | bg10600 | unknown |
| ywtd | bg12535 | unknown; similar to murein hydrolase |

Tables 10 lists the genes that were uniquely downregulated due to treatment with the cell wall inhibitors cephalothin or vancomycin. The genes downregulated due to treatment with cephalothin or vancomycin were not affected by any of the other antibiotics tested herein.

**Table 10**

| **Gene** | **Accession number** | **Description** |
|---|---|---|
| ctaa | bg10213 | cytochrome caa3 oxidase (required for biosynthesis) |
| ctab | bg10214 | cytochrome caa3 oxidase (assembly factor) |
| cyda | bg11925 | cytochrome bd ubiquinol oxidase (subunit I) |
| feub | bg10836 | iron-uptake system (integral membrane protein) |
| lctp | bg12001 | L-lactate permease |
| nfra | bg10589 | FMN-containing NADPH-linked nitro/flavin reductase |
| rpmg a | bg14180 | possible ribosomal protein L33 |
| srfac | bg10170 | surfactin synthetase / competence |
| tkt | bg11247 | transketolase |
| yetg | bg12863 | unknown; similar to unknown proteins |
| yloh | bg13387 | unknown; similar to unknown proteins |
| ypib | bg11497 | unknown; similar to unknown proteins |
| yrbf | bg13785 | unknown; similar to unknown proteins |
| ywdd | bg10600 | unknown |

Table 11 lists the genes that were downregulated due to treatment with all four protein synthesis inhibitors.

**Table 11**

| **Gene** | **Accession number** | **Description** |
|---|---|---|
| acpa | bg11536 | acyl carrier protein |
| ahpf | bg11204 | alkyl hydroperoxide reductase (large subunit) / NADH dehydrogenase |
| appd | bg11085 | oligopeptide ABC transporter (ATP-binding protein) |
| clpx | bg11387 | ATP-dependent Clp protease ATP-binding subunit (class III heat-shock protein) |
| glya | bg10944 | serine hydroxymethyltransferase |
| mntb | bg13852 | manganese ABC transporter (ATP-binding protein) |
| mntb | bg13852 | manganese ABC transporter (ATP-binding protein) |
| mntb | bg13852 | manganese ABC transporter (ATP-binding protein) |
| mntb | bg13852 | manganese ABC transporter (ATP-binding protein) |
| mntd | bg13854 | manganese ABC transporter |
| mntd | bg13854 | manganese ABC transporter |
| purd | bg10711 | phosphoribosylglycinamide synthetase |
| resa | bg10531 | essential protein similar to cytochrome c biogenesis protein |
| resa | bg10531 | essential protein similar to cytochrome c biogenesis protein |
| rplw | bg11221 | ribosomal protein L23 |
| rpmj | bg11042 | ribosomal protein L36 (ribosomal protein B) |
| rpob | bg10728 | RNA polymerase (beta subunit) |
| veg | bg10107 | function unknown |
| ycgo | bg12013 | unknown; similar to proline permease |
| yetj | bg12866 | unknown; similar to unknown proteins |
| yfha | bg12876 | unknown; similar to iron(III) dicitrate transport permease |
| ykuc | bg13287 | unknown; similar to macrolide-efflux protein |
| ykuj | bg13294 | unknown |
| ylag | bg13344 | unknown; similar to GTP-binding elongation factor |
| ylag | bg13344 | unknown; similar to GTP-binding elongation factor |
| ylag | bg13344 | unknown; similar to GTP-binding elongation factor |
| ylag | bg13344 | unknown; similar to GTP-binding elongation factor |
| ylag | bg13344 | unknown; similar to GTP-binding elongation factor |
| ylag | bg13344 | unknown; similar to GTP-binding elongation factor |
| ynef | bg11249 | unknown; similar to unknown proteins |
| yqgx | bg11691 | unknown; similar to unknown proteins |
| yqgx | bg11691 | unknown; similar to unknown proteins |
| ytip | bg13864 | unknown; similar to unknown proteins |
| ytqb | bg13909 | unknown; similar to unknown proteins |
| ytqb | bg13909 | unknown; similar to unknown proteins |
| yurv | bg14008 | unknown; similar to NifU protein homolog |
| ywza | bg14162 | unknown; similar to unknown proteins from B. subtilis |
| ywza | bg14162 | unknown; similar to unknown proteins from B. subtilis |

Tables 12 lists the genes that were uniquely downregulated due to treatment with the protein synthesis inhibitors, chloramphenicol, erythromycin, gentamicin and streptomycin. The genes were downregulated due to treatment with the protein synthesis inhibitors but not due to any of the other antibiotics.

**Table 12**

| **Gene** | **Accession number** | **Description** |
|---|---|---|
| appd | bg11085 | oligopeptide ABC transporter (ATP-binding protein) |
| atpd | bg10821 | ATP synthase (subunit beta) |
| eno | bg10899 | enolase |
| luxs | bg13866 | probable autoinducer-2 production protein |
| mlpa | bg10779 | mitochondrial processing peptidase-like |
| mnta | bg13851 | manganese ABC transporter (membrane protein) |
| mntd | bg13854 | manganese ABC transporter |
| opuab | bg11371 | glycine betaine ABC transporter (permease) |
| purd | bg10711 | phosphoribosylglycinamide synthetase |
| resa | bg10531 | essential protein similar to cytochrome c biogenesis protein |
| rpob | bg10728 | RNA polymerase (beta subunit) |
| ycgo | bg12013 | unknown; similar to proline permease |
| yfha | bg12876 | unknown; similar to iron(III) dicitrate transport permease |
| ykoe | bg13250 | unknown |
| ykuj | bg13294 | unknown |
| ylag | bg13344 | unknown; similar to GTP-binding elongation factor |
| ynef | bg11249 | unknown; similar to unknown proteins |
| ytip | bg13864 | unknown; similar to unknown proteins |
| ytqb | bg13909 | unknown; similar to unknown proteins |
| yuru | bg14007 | unknown; similar to unknown proteins |
| yurv | bg14008 | unknown; similar to NifU protein homolog |
| yusv | bg14034 | unknown; similar to iron(III) dicitrate transport permease |

Table 13 lists the genes that were uniquely down regulated due to treatment with the aminoglycoside protein synthesis inhibitor streptomycin or gentamicin. These genes are downregulated due to treatment with streptomycin or gentamicin but not due to treatment with ciprofloxacin, norfloxacin, cephalothin, vancomycin or trimethoprim. Only a single gene, *yrbd* (bg13784), was uniquely upregulated due to treatment with streptomycin or gentamicin.

**Table 13**

| **Gene** | **Accession number** | **Description** |
|---|---|---|
| alat | bg12362 | putative alanine transaminase |
| mrpb | bg12356 | multiple resistance and pH homeostasis |
| pssa | bg11012 | phosphatidylserine synthase |
| secdf | bg12672 | protein-export membrane protein |
| xepa | bg10959 | PBSX prophage lytic exoenzyme |
| yfln | bg12949 | unknown; similar to unknown proteins |
| yisx | bg13103 | unknown; similar to unknown proteins |
| ymcb | bg13418 | unknown; similar to unknown proteins |
| yoje | bg13557 | unknown; similar to unknown proteins |
| yqzd | bg13770 | unknown |
| ywdj | bg10606 | unknown; similar to unknown proteins |
| ywhc | bg12457 | unknown; similar to unknown proteins |
| yycb | bg10007 | unknown; similar to ABC transporter (permease) |

The lists can also be analyzed using clustering tools as described in Example 7. Genes that are clustered together due to their expression patterns are useful in studies for determining the function of y-genes, which are genes whose function is not known, and also to identify regulons that might be affected by treatment with a particular antibiotic or class of antibiotics.

Table 14 lists genes whose expression pattern was similar due to treatment with erythromycin. These genes were upregulated at 15 minutes at most, if not all, concentrations and downregulated at 30 or 60 minutes.

**Table 14**

| **Gene** | **Accession number** |
|---|---|
| apt | bg12563 |
| atpa | bg10819 |
| atpe | bg10816 |
| atpg | bg10820 |
| mdr | bg12002 |
| pbug | bg12811 |
| pbux | bg11080 |
| rpla | bg10164 |
| rplb | bg11217 |
| rplc | bg11218 |
| rpld | bg11219 |
| rplk | bg10163 |
| rpll | bg10726 |
| rplm | bg11970 |
| rplu | bg10333 |
| rplv | bg11078 |
| rplw | bg11221 |
| rpme | bg10417 |
| rpmga | bg14180 |
| rpmj | bg11042 |
| rpob | bg10728 |
| rpsc | bg19005 |
| rpsd | bg10372 |
| rpsj | bg19008 |
| rpsl | bg19009 |
| rpso | bg19010 |
| rpsq | bg10757 |
| rpss | bg19011 |
| sped | bg13832 |
| spovg | bg10112 |
| ycek | bg12775 |
| ycgo | bg12013 |
| yczg | bg12781 |
| yczi | bg12783 |
| ydah | bg12055 |
| ydbi | bg12076 |
| ydbl | bg12079 |
| yebc | bg12812 |
| yerq | bg12843 |
| yetj | bg12866 |
| yetk | bg12867 |
| yfna | bg12972 |
| yhdh | bg13014 |
| ykuk | bg13295 |
| ylbn | bg13366 |
| yloh | bg13387 |
| ynef | bg11249 |
| yqdb | bg11512 |
| yqhl | bg11700 |
| ysda | bg12315 |
| ytip | bg13864 |
| yuif | bg13971 |
| yvsh | bg14154 |
| ywbg | bg10568 |
| ywbh | bg10569 |
| ywdk | bg10607 |
| ywpb | bg12496 |
| yxja | bg11150 |
| zur | bg11668 |

Table 15 are genes classified as transport/binding proteins whose expression were affected by treatment with the protein synthesis inhibitors.

**Table 15**

| **Gene** | **Accession number** |
|---|---|
| *feuB* | bg10836 |
| *glpF* | bg10186 |
| *gltT* | bg 12595 |
| *manP* | bg 13176 |
| *nagP* | bg12941 |
| *pbuG* | bg12811 |
| *pbuX* | bg11080 |
| *rbsB* | bg10881 |
| *rbsD* | bg10878 |

Table 16 are genes classified as being involved in carbohydrate metabolism whose expression were affected by treatment with one of the protein synthesis inhibitors.

**Table 16**

| **Gene** | **Accession number** |
|---|---|
| *citB* | bg10478 |
| *glpK* | bg10187 |
| *icd* | bg10856 |
| *iolB* | bg11118 |
| *iolE* | bg11121 |
| *odhA* | bg10272 |
| *rbsK* | bg10877 |
| *sdhC* | bg10351 |
| *ydjE* | bg12796 |
| *ydjL* | bg12803 |

The expression levels of many of the genes classified as being involved in protein synthesis were affected by treatment with a protein synthesis inhibitor. Table 17 lists these genes.

**Table 17**

| **Gene** | **Accession number** |
|---|---|
| *rplB* | bg11217 |
| *rplE* | bg10760 |
| *rplF* | bg11408 |
| *rplJ* | bg11220 |
| *rplL* | bg10726 |
| *rplM* | bg11970 |
| *rplO* | bg10444 |
| *rplQ* | bg11041 |
| *rplR* | bg11409 |
| *rplU* | bg10333 |
| *rplV* | bg11078 |
| *rplW* | bg11221 |
| *rpmC* | bg10756 |
| *rpmD* | bg10443 |
| *rpmJ* | bg11042 |
| *rpsC* | bg19005 |
| *rpsE* | bg10442 |
| *rpsH* | bg10762 |
| *rpsl* | bg19007 |
| *rpsJ* | bg19008 |
| *rpsM* | bg10730 |
| *rpsN* | bg 10761 |
| *rpsP* | bg10831 |
| *rpsQ* | bg10757 |
| *rpsS* | bg19011 |

Table 18 are genes that were highly expressed (greater than 3 fold induction) in response to various concentrations of chloramphenicol, erythromycin, or gentamicin.

**Table 18**

| **Gene** | **Accession number** |
|---|---|
| *dctP* | bg12075 |
| *ysbA* | bg12311 |
| *ysbB* | bg12312 |

Table 19 lists genes highly expressed due to treatment with one of the protein synthesis inhibitors.

**Table 19**

| **Gene** | **Accession number** | |
|---|---|---|
| *clpP* | bg19016 | in gentamicin |
| *dppB* | bg10843 | in erythromycin |
| *gapB* | bg12592 | in chloramphenicol |
| *mcpB* | bg10859 | in chloramphenicol |
| *rbsB* | bg10881 | in chloramphenicol |
| *rpsF* | bg10049 | in erythromycin |
| *ycnB* | bg12038 | in erythromycin |
| *yheH* | bg13040 | in chloramphenicol |
| *yhel* | bg13041 | in chloramphenicol |
| *yolF* | bg13584 | in erythromycin |
| *yonS* | bg13629 | in erythromycin |
| *yrzl* | bg13819 | in chloramphenicol |
| *ytiP* | bg13864 | in erythromycin |
| *yvsH* | bg14154 | in erythromycin |
| *yxiE* | bg11134 | in gentamicin |

Table 20 are genes specifically upregulated due to treatment with chloramphenicol or erythromycin.

**Table 20**

| **Gene** | **Accession number** |
|---|---|
| *cspB* | bg10824 |
| *cspD* | bg11531 |
| *purB* | bg10702 |
| *purC* | bg10703 |
| *purD* | bg10711 |
| *purE* | bg10700 |
| *purM* | bg10708 |
| *pyrAA* | bg10715 |
| *pyrAB* | bg10716 |
| *pyrB* | bg10713 |
| *pyrC* | bg10714 |
| *pyrE* | bg10720 |
| *pyrF* | bg10719 |
| *xpt* | bg11079 |

Table 21 is a list of genes whose expression is effected by treatment with rifampin.

**Table 21**

| **Gene** | **Accession number** |
|---|---|
| adk | bg10446 |
| citz | bg10855 |
| cspd | bg11531 |
| eno | bg10899 |
| gapa | bg10827 |
| glna | bg10425 |
| glya | bg10944 |
| groel | bg10423 |
| gtab | bg10402 |
| gyra | bg10071 |
| hag | bg10655 |
| pgk | bg11062 |
| rpll | bg10726 |
| rpoa | bg10732 |
| rsbv | bg10733 |
| soda | bg11676 |
| srfad | bg10171 |
| succ | bg12680 |
| yjbg | bg13136 |
| ylba | bg13353 |
| yocj | bg13523 |
| yodc | bg13532 |
| yugu | bg12373 |
| yurp | bg14002 |
| yvct | bg12409 |
| cith | bg11146 |
| clpc | bg10148 |
| clpp | bg19016 |
| dps | bg12584 |
| gsib | bg10826 |
| ilvc | bg10672 |
| kata | bg10849 |
| kate | bg11102 |
| mrga | bg10864 |
| rpob | bg10728 |
| rsbw | bg10734 |
| sucd | bg12681 |
| yfkm | bg12929 |
| ykwc | bg13328 |
| ytxh | bg10975 |
| yvyd | bg10740 |
| sigb | bg10735 |
| bmru | bg10302 |
| gspa | bg10558 |
| tufa | bg11056 |

### Example 21: Potential Targets for Drug Development

The gene lists described in Example 19 as well as the Tables in Example 20 were mined for potential drug targets. Targets were chosen based on several criteria including the following: (1) the gene is upregulated or downregulated at a low concentration and an early timepoint after antibiotic addition since these differences in gene expression are more likely to be due to a primary response (for instance the genes in Table 14 whose expression is induced at an early timepoint after the addition of erythromycin), or (2) the gene is uniquely upregulated or downregulated due to treatment with antibiotics from a given mode of action class (for instance the genes listed in Table 6 and 7 which are uniquely upregulated or downregulated due to treatment with the DNA gyrase inhibitors).

An ideal target is one that has a *Staphylococcus aureus* and/or *Streptococcus pneumoniae* homolog. This can be determined by searching the predicted protein sequence for the *Bacillus subtilis* gene against all the predicted proteins of *Staphylococcus aureus* or *Streptococcus pneumoniae* using the Smith-Waterman algorithm. A good target candidate would also ideally not have a human homolog; for a given *Bacillus subtilis* gene this can be determined by doing a search using the BLAST algorithm comparing the predicted protein sequence of the gene to the entire human database translated in all six reading frames. This provides results for potential homologs listing the percent identity and an e value which provide information on how likely the human gene is a homolog to the *Bacillus subtilis* gene. A threshold level for a gene to be considered a significant human homolog was adopted, in that the percent identity must be greater than 20% and/or the e value must be less than e⁻⁴. Furthermore, the potential targets must be genes that are known to be essential for growth or for which it is not known if they are essential. All genes that are known to be non-essential are not considered valid targets. Table 22 lists potential targets along with their *Staphylococcus aureus* and/or *Streptococcus pneumoniae* homologs.

**Table 22**

| **Gene** | **Accession number** | **Staphylococcus gene** | **Streptococcus gene(s)** |
|---|---|---|---|
| dctp | BG12075 | none | none |
| resa | BG10531 | none | SP0659 & SP1000 |
| ybxf | BG11365 | SA0502 | SP0555 |
| ybxg | BG11505 | SA2109 | none |
| ycef | BG12770 | none | none |
| yceh | BG12772 | SA1238 | none |
| ycek | BG12775 | none | none |
| yclo | BG12035 | SA0689 | SP1870 |
| ycsa | BG11222 | none | none |
| ycsn | BG11235 | SA0643 | SP0791 |
| yczi | BG12783 | none | none |
| ydbi | BG12076 | none | none |
| ydce | BG12092 | SA1873 | none |
| ydeh | BG12135 | SA0362 | none |
| ydfq | BG12164 | SA2324 | none |
| ydip | BG12788 | SA0569 | SP0569 & SP1336 |
| ydpf | BG12163 | SA1117 | SP0908 |
| yebc | BG12812 | none | none |
| yerq | BG12843 | SA1714 & SA0681 | SP1045 |
| yetj | BG12866 | SA0621 | SP1972 |
| yetk | BG12867 | none | none |
| yfms | BG12970 | none | none |
| yfmt | BG12971 | none | SP1119 |
| yhdh | BG13014 | SA0417 | SP0737 & SP0738 |
| yheh | BG13040 | none | SP1308 & SP1839 |
| yhei | BG13041 | none | SP1840 |
| yhja | BG13068 | none | none |
| ykuk | BG13295 | none | none |
| ykzg | BG13335 | SA0941 | SP0122 |
| ylbn | BG13366 | SA0975 | SP1280 |
| ynea | BG11820 | none | none |
| ynef | BG11249 | SA1178 | SP1802 |
| yolf | BG13584 | none | none |
| yqdb | BG11512 | none | none |
| yqei | BG11637 | SA1423 | SP1748 |
| yqej | BG11638 | SA1422 | SP1747 |
| yqhl | BG 11700 | SA0042, SA0044, SA1364, SA1578 | SP0678 |
| yrbf | BG13785 | SA1464 | SP2029 |
| ysba | BG12311 | none | none |
| ysbb | BG12312 | none | none |
| yuif | BG13971 | none | none |
| yvce | BG11023 | SA1077 | SP0217 & SP2216 |
| ywdi | BG10605 | none | none |
| ywdj | BG10606 | none | none |
| ywdk | BG10607 | none | none |
| ywpb | BG12498 | SA1901 | SP0424 |
| yxja | BG11150 | none | none |
| yxie | BG11134 | SA1528 & SA1532 | SP1996 |
| yyaa | BG10057 | SA0348 & SA2498 | SP2240 |

### Example 22: Knockout of yerQ

For those potential targets for which there is no data on whether the gene was essential or not for *Bacillus subtilis* growth, the first step in target validation was to determine if deletion of the gene was essential or not. One of the potential targets identified was *yerQ.*

The *yerQ* coding region is 903 bp long, the central 500 bp of which have been deleted in Δ*yerQ*. Δ*yerQ* was created by Splicing by Overlap Extension (SOE). The upstream and the downstream portions of the *yerQ* gene were PCR amplified from *Bacillus subtilis* strain BGSC1A2 chromosomal DNA using primer 1 and primer 2 for the upstream end and primer 3 and primer 4 for the downstream end. Primer 2 and primer 3 were designed to complement each other. Convenient restriction sites were added at the 5' end of each primers, in particular *Bam*HI in primer 1, *Hind*III in primers 2 and 3, and *Asp*718 in primer 4. The primers have the following sequences:
Primer 1:
   5'-CGGGATCCAGCTTGTTGAAAAACCCTCGC-3' (SEQ ID NO: 1)
Primer 2 :
   5'-TGCTTTCTTTAGTATCATCAAAGCTTCCGCTTCCTTGGCAGCGTGTGT-3' (SEQ ID NO: 2)
Primer 3:
   5'-ACACACGCTGCCAAGGAAGCGGAAGCTTTGATGATACTAAAGAAAGCA-3' (SEQ ID NO: 3)
Primer 4:
   5'-GGGGTACCAGCGTGTAGGCAAACCTTCGCA-3' (SEQ 10 NO: 4)

The chromosomal DNA extraction from *Bacillus subtilis* strain BGSC1A2 and the two PCR reactions were performed using the REDExtract-N-Amp™ Plant PCR Kit (SIGMA, St. Louis, MO) according to the manufacturer's instructions. Amplification reactions were conducted in a RoboCycler 40 Temperature Cycler (Stratagene, Inc, La Jolla, CA) programmed for 1 cycle at 96°C for 10 minutes; 30 cycles each at 96°C for 1 minute, 55°C for 1 minute, and 72°C for 2 minutes; and a final cycle at 72°C for 7 minutes. Reaction products were analyzed by agarose gel electrophoresis using a 0.8% agarose-25 mM Tris base-25 mM borate-0.5 mM disodium EDTA buffer (0.5X TBE) gel. Then the products were gel purified using a QIAquick Gel Extraction Kit (QIAGEN, Inc., Valencia, CA) according to the manufacturer's instructions.

The two purified PCR products were used as template in a third amplification reaction with the following composition: about 5 ng of the upstream and downstream *yerQ* bands, 0.5 µM each of primers 1 and 4, 200 µM each of dATP, dCTP, dGTP, and dTTP, 1X PCR Buffer II (Applied Biosystems, Inc., Foster City, CA) with 1.5 mM MgCl₂, and 2.5 units of AmpliTaq Gold™ DNA polymerase (Applied Biosystems, Inc., Foster City, CA). The reactions were performed in a RoboCycler 40 Temperature Cycler programmed for 1 cycle at 96°C for 10 minutes; 30 cycles each at 96°C for 1 minute, 55°C for 1 minute, and 72°C for 2 minutes; and 1 cycle at 72°C for 7 minutes. The PCR product was visualized using a 0.8% agarose-0.5X TBE gel, and purified using the QIAquick Gel Extraction Kit (QIAGEN, Inc., Valencia, CA).

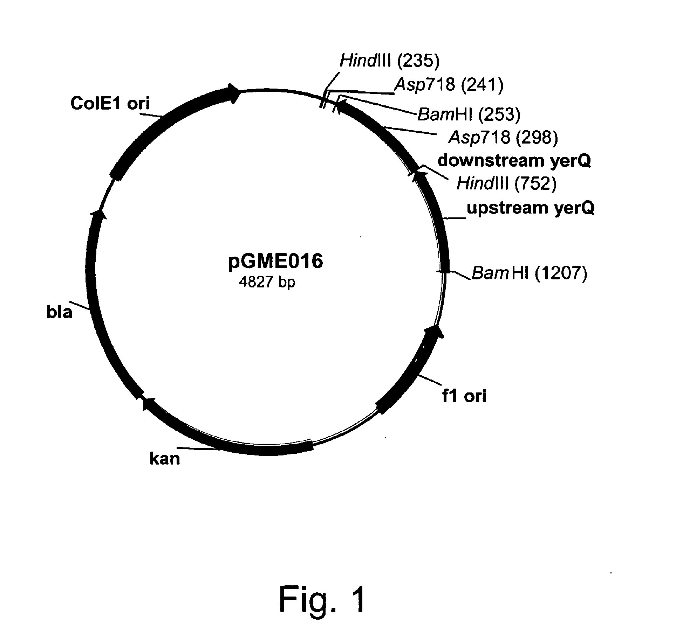

The resulting fragment comprising the deleted *yerQ* was cloned into pCR2.1-TOPO vector using the TA-TOPO Cloning Kit and transformed into *E. coli* OneShot™ Top10 cells according to the manufacturer's instructions (Invitrogen, Inc., Carlsbad, CA). Transformants were selected on Yeast-Tryptone (2X YT) agar plates supplemented with 100 µg of ampicillin per ml, and grown at 37°C for about 12 hours. Plasmid DNA from several transformants was isolated using the QIAGEN Plasmid Mini Purification protocol, according to the manufacturer's instructions (QIAGEN, Inc., Valencia, CA) and checked by PCR amplification using the M13(-20) forward and M13(-24) reverse primers (Invitrogen, Inc, Carlsbad, CA). The amplification reactions (50 µl) were composed of approximately 25 ng of plasmid DNA, 0.5 µM of each primers M13(-20)forward and M13(-24) reverse, 200 µM each of dATP, dCTP, dGTP, and dTTP, 1X PCR Buffer II (Applied Biosystems, Inc., Foster City, CA) with 1.5 mM MgCl₂, and 2.5 units of AmpliTaq Gold™ DNA polymerase (Applied Biosystems, Inc., Foster City, CA). The reactions were performed in a RoboCycler 40 Temperature Cycler programmed for 1 cycle at 96°C for 10 minutes; 30 cycles each at 96°C for 1 minute, 55°C for 1 minute, and 72°C for 2 minutes; and 1 cycle at 72°C for 7 minutes. The PCR product was visualized using a 0.8% agarose-0.5X TBE gel. The resulting plasmid was called pGME016 (Figure 1). The orientation of the Δ*yerQ* cloned into pGME016 was determined by digestion of the plasmid using restriction enzymes *Bam*HI and *Hind*III.

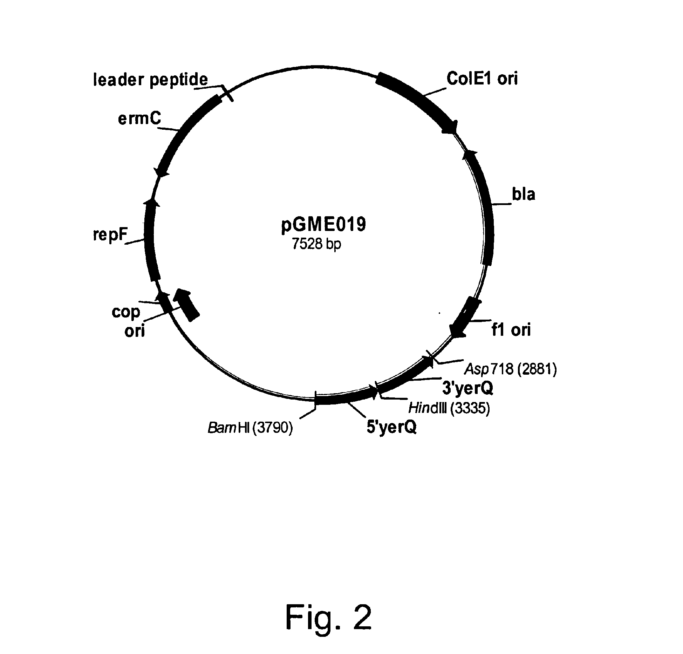

A fragment of 909 bp from pGME016, bearing the Δ*yerQ* construct, and the vector fragment of 6619 bp from pNNB194 (pSK+/pE194; US Patent No. 5,958,728), obtained after digestion with *Asp*718 and *Bam*HI, were isolated from a 0.8% agarose-0.5X TBE gel using the QIAquick Gel Extraction Kit (QIAGEN, Inc., Valencia, CA) and ligated together. The ligation reaction (20 µl) was performed using the Rapid DNA Ligation Kit (Roche Applied Science, Indianapolis, IN) according to the manufacturer's instructions. A 10 µl volume of the ligation reaction was used to transform *E. coli* Epicurian Coli XL10-Gold Ultracompetent cells according to the manufacturer's instructions (Stratagene, Inc., La Jolla, CA). Transformants were selected on 2X YT agar plates supplemented with 100 µg of ampicillin per ml and grown overnight at 37°C. Plasmid DNA from several transformants was isolated using the QIAGEN Plasmid Mini Purification protocol (QIAGEN, Inc., Valencia, CA), and checked by digestion with restriction enzymes *Asp*718 and *Bam*HI. This plasmid was called pGME019 (Figure 2).

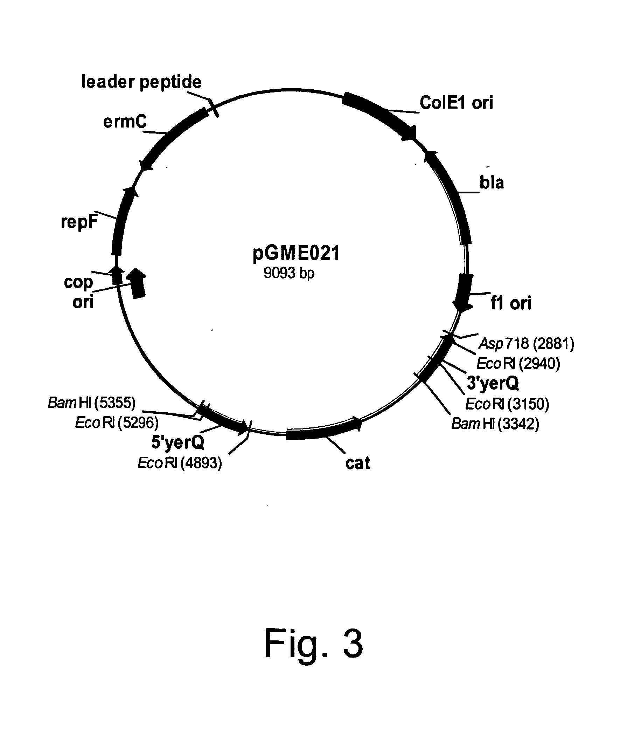

pGME019 was digested with *Hind*III; the digested plasmid was treated with T4 polymerase to generate blunt ends according to the manufacturer's instruction (Roche Applied Science, Indianapolis, IN); and the ends were dephosphorylated using Shrimp Alkaline Phosphatase, SAP (Roche Applied Science, Indianapolis, IN), following the protocol provided by the manufacturer. Furthermore plasmid pECC1 (Youngman et al., 1984, Plasmid 12: 1-9) was digested using restriction enzyme *Sma*I and the 1561 bp fragment bearing the *cat* gene (conferring chloramphenicol resistance) was gel purified and ligated with the dephosphorylated pGME019 using the Rapid DNA Ligation Kit (Roche Applied Science, Indianapolis, IN). A 10 µl volume of the ligation reaction was used to transform *E. coli* Epicurian Coli XL10-Gold Ultracompetent cells (Stratagene, Inc., La Jolla, CA) and the transformants were selected on 2X YT agar plates supplemented with 100 µg of ampicillin per ml and grown overnight at 37°C. Eighteen transformants were selected and grown overnight at 37°C for plasmid mini extraction following the QIAGEN Plasmid Mini Purification protocol. The recovered plasmid DNA was digested with *Bam*HI and the digestion reaction results were visualized using a 0.8% agarose-0.5X TBE gel. Thi construct was designated pGME021 (Figure 3).

Plasmid pGME021 was digested with *Sca*I and *Pst*I. The 4051 bp fragment containing the construct *5'yerQ-cat-3'yerQ* was gel purified from a 0.8% agarose-0.5X TBE gel using the QIAquick Gel Extraction Kit, and transformed into *Bacillus subtilis* strain BGSC1A2 competent cells (Anagnostopoulos and Spizizen, 1961, Journal of Bacteriology 81: 741-746) in presence of 0.2 µg of chloramphenicol per ml for induction of the *cat* gene. Transformants were selected on Tryptose blood agar base (TBAB; Difco, Detroit MI) plates containing 5 µg of chloramphenicol per ml, and grown at 34°C for about 14 hours. The introduction of a linear DNA fragment and the selective growth on chloramphenicol led to the substitution of the chromosomal *yerQ* with the construct 5'*yerQ-cat-*3'*yerQ* by homologous recombination at the *yerQ* locus. The obtained clones showed a different colony morphology compared to the wild type *Bacillus subtilis* strain BGSC1A2 when grown on TBAB plates containing 5 µg of chloramphenicol per ml. In particular, some of the clones developed a translucent appearance on plate, while the wild type formed solid colonies. Four of these clones were checked by two PCRs, using primer 1 and primer 6 in the first and primer 5 and primer 4 in the second reaction. Primers 5 and 6 bind within the *cat* gene sequence.
Primer 5 :
   5'-TAGACAATTGGAAGAGAAAAGAGATA-3' (SEQ ID NO: 5)
Primer 6 :
   5'-ATGCATGGAGCTGTAATATAAAAACC-3' (SEQ ID NO: 6)

The chromosomal DNA extraction from the analyzed clones and the two PCR reactions were performed using the REDExtract-N-Amp™ Plant PCR Kit (SIGMA, Saint Louis, Missouri) according to the manufacturer's instructions. Amplification reactions were conducted in a RoboCycler 40 Temperature Cycler programmed for 1 cycle at 96°C for 10 minutes; 30 cycles each at 96°C for 1 minute, 55°C for 1 minute, and 72°C for 2 minutes, followed by 1 cycle at 72°C for 7 minutes. The PCR product was visualized using a 0.8% agarose-0.5X TBE gel. Only one of the analyzed clones gave a PCR product with both primer pairs. Furthermore, this clone maintained a translucent appearance when streaked on TBAB plates containing 5 µg of chloramphenicol per ml or on simple LB (Luria-Bertani) plates.

This data suggests that *yerQ* is very important for growth if not essential.

Since *yerQ* appeared to be essential and was chosen based on the fact that its expression was upregulated due to treatment with both ciprofloxacin and chloramphenicol, genes whose expression pattern was similar to *yerQ* in the presence of ciprofloxacin or chloramphenicol were identified. The lists of upregulated genes following treatment with ciprofloxacin or chloramphenicol were clustered based on expression pattern using a complex correlation (smooth) in the GeneSpring software package. In Table 23 below are the genes whose expression pattern is similar to *yerQ* due to treatment with ciprofloxacin or chloramphenicol; only genes that were common due to treatment with ciprofloxacin or chloramphenicol are included. These genes represent potential targets.

**Table 23**

| | | |
|---|---|---|
| **<Complex Correlation (smooth correlation) with *yerQ* from CHL and CIP experiments** | | |

| **Gene Name** | **BG #** | **Function** |
|---|---|---|
| abh | bg10988 | regulation of transition state genes |
| cina | bg11374 | |
| cspc | bg11024 | |
| cspr | bg11802 | |
| ctaa | bg10213 | required for biosynthesis of cytochrome caa3 oxidase |
| ctab | bg10214 | |
| def | bg11933 | |
| dltd | bg10548 | D-alanine esterification of lipoteichoic acid and wall teichoic acid (D-alanine transfer from undecaprenol-P to the poly(glycerophosphate) chain of LTA) |
| ftse | bg12590 | |
| gsib | bg10826 | |
| hutp | bg10666 | positive regulation of the histidine utilization operon (hutPHUIGM) |
| lyte | bg11406 | cell wall lytic activity |
| mntd | bg13854 | manganese uptake |
| mnth | bg12065 | manganese uptake |
| moad | bg12619 | |
| pyrr | bg10712 | attenuation (antitermination) of the pyrimidine operon (pyrPBCADFE) in the presence of UMP |
| qoxd | bg10586 | |
| rpmh | bg10064 | |
| rpsd | bg10372 | |
| tago | bg12684 | teichoic acid linkage unit synthesis (synthesis of undecaprenylpyrophosphate-N-aetylglucosamine) |
| ybxg | bg11505 | unknown |
| ycbp | bg11171 | unknown |
| ycek | bg12775 | unknown |
| yddt | bg12127 | unknown |
| yebc | bg12812 | unknown |
| yerq | bg12843 | unknown |
| yfiw | bg12899 | unknown |
| yhai | bg12985 | unknown |
| yhcu | bg11599 | unknown |
| yhej | bg13042 | unknown |
| yhjn | bg13080 | unknown |
| yitw | bg12247 | unknown |
| yjbd | bg13133 | unknown |
| yjcf | bg13159 | unknown |
| yjzc | bg13223 | unknown |
| yjzd | bg13224 | unknown |
| ykok | bg13256 | unknown |
| ykox | bg13267 | unknown |
| ykrm | bg13275 | unknown |
| ykuc | bg13287 | unknown |
| ykza | bg19021 | unknown |
| ylbb | bg13354 | unknown |
| ylbf | bg13358 | unknown |
| yloc | bg13385 | unknown |
| yloh | bg13387 | unknown |
| ymca | bg13417 | unknown |
| ymfm | bg13433 | unknown |
| ynab | bg12254 | unknown |
| ynej | bg11251 | unknown |
| yner | bg11825 | unknown |
| ynet | bg11827 | unknown |
| yngc | bg13454 | unknown |
| yoch | bg13521 | unknown |
| yoml | bg13599 | unknown |
| yops | bg13652 | unknown |
| yoza | bg13748 | unknown |
| yozc | bg13750 | unknown |
| ypdc | bg11438 | unknown |
| yqey | bg11649 | unknown |
| yqgs | bg11686 | unknown |
| yqzd | bg13770 | unknown |
| ysfc | bg12320 | unknown |
| ysfd | bg12321 | unknown |
| ytkd | bg13869 | unknown |
| ytxj | bg10373 | unknown |
| yufk | bg12346 | unknown |
| yuib | bg13967 | unknown |
| yusj | bg14022 | unknown |
| yuti | bg14045 | unknown |
| yuza | bg14050 | unknown |
| yvce | bg11023 | unknown |
| yvgj | bg14092 | unknown |
| yvgt | bg14102 | unknown |
| ywla | bg10936 | unknown |
| ywnc | bg12481 | unknown |
| yxis | bg11148 | unknown |
| yxja | bg11150 | unknown |
| yxjb | bg11151 | unknown |

### Example 23: Responsive Promoter Construct

The data generated from DNA microarray analysis of *Bacillus subtilis* cultures treated with antibiotics as described in Example 2 can also be used to identify genes whose expression pattern can be used as a reporter for a particular class of antibiotics. The promoter for these genes is fused to a reporter protein, such as green fluorescent protein, or any other reporter that can be assayed to create a series of constructs in which expression of the reporter genes is under the control of the "antibiotic" inducible promoter. Such constructs can then be introduced into *Bacillus subtilis* and the resulting strain, when treated with antibiotics, assayed for expression of the reporter protein.

A good example of inducible promoters that could be used as reporters for a particular antibiotic or class of antibiotics are the genes listed in Tables 4-23 or a subset of these genes. An ideal reporter would be one whose expression due to drug treatment is induced or repressed at a ratio greater than two in comparison to an untreated culture.

### Example 24: Reporter Strains

The constructs described in Example 23 could be used to generate a series of *Bacillus subtilis* strains each containing a single promoter/reporter protein construct. These strains could be arrayed in a microtiter plate with each well containing a single strain. The strains could be grown to exponential phase in MHB as described in Example 2 above and then a compound with antimicrobial activity could be added and over time the plate could be assayed for the expression of the reporter protein. Based on which reporter strains gave significantly more or less expression of the reporter protein one might be able to determine if a new antimicrobial compound had a mode of action similar to known antibiotics.

The invention described and claimed herein is not to be limited in scope by the specific embodiments herein disclosed, since these embodiments are intended as illustrations of several aspects of the invention. Indeed, various modifications of the invention in addition to those shown and described herein will become apparent to those skilled in the art from the foregoing description.

## Claims

1. A method for determining the mode of action of an antimicrobial compound, comprising:
(a) detecting hybridization complexes formed by contacting at least one nucleic acid sample, obtained by culturing *Bacillus subtilis* cells in the presence of at least one sub-inhibitory amount of an antimicrobial compound having an unknown mode of action, with a plurality of nucleic acid sequences corresponding to genes of the *Bacillus subtilis* cells, wherein the presence, absence or change in the amount of the hybridization complexes detected, compared with hybridization complexes formed between the plurality of nucleic acid sequences and a second nucleic acid sample obtained from the *Bacillus subtilis* cells cultured in the absence or presence of a standard compound having a known mode of action selected from the group consisting of cephalothin, chloramphenicol, ciprofloxacin, erythromycin, gentamicin, gramicidin A, nalidixic acid, norfloxacin, novobiocin, rifampin, streptomycin, trimethoprim, and vancomycin, is indicative of the similarity or dissimilarity of the mode of actions of the antimicrobial compound and the standard compound; and
(b) assigning a mode of action for the antimicrobial compound based on the similarity or dissimilarity of values assigned to the hybridization complexes detected in (a) based on the relative amount of hybridization to a second set of hybridization values assigned to the hybridization complexes formed from the second nucleic acid sample;
wherein the sub-inhibitory amount is based on the minimal inhibitory concentration (MIC) of the antimicrobial compound against a bacterium and cultivation of the bacterium at one or more concentrations below the MIC, wherein MIC is defined as that concentration of an antimicrobial compound resulting in no visible growth of the organism.

2. The method of claim 1, wherein the step in (b) comprises subjecting the set of values to a program for analysis.

3. The method of claim 2, wherein the program for analysis comprises a computer algorithm.

4. The method of claim 1, wherein the antimicrobial compound is a member of the class of antimicrobial compounds that inhibit cell wall synthesis, interfere with the cell membrane, inhibit protein synthesis, inhibit topoisomerase activity, inhibit RNA synthesis, or is a competitive inhibitor.

5. The method of claim 1, wherein the plurality of sequences are obtained from an organism different from *Bacillus subtilis.*

6. The method of claim 1, wherein the plurality of sequences are obtained from *Bacillus subtilis.*

7. The method of claim 1, wherein the plurality of sequences correspond to less than about 75% of the genome of the *Bacillus subtilis* cells.

8. The method of claim 1, wherein the plurality of sequences correspond to less than about 50% of the genome of the *Bacillus subtilis* cells.

9. The method of claim 1, wherein the plurality of sequences correspond to less than about 25% of the genome of the *Bacillus subtilis* cells.

10. The method of claim 1, wherein the plurality of sequences correspond to less than about 10% of the genome of the *Bacillus subtilis* cells.

11. The method of claim 1, wherein the plurality of sequences correspond to less than about 5% of the genome of the *Bacillus subtilis* cells.

12. The method of claim 1, wherein the plurality of sequences correspond to less than about 2% of the genome of the *Bacillus subtilis* cells.

13. The method of claim 1, wherein the plurality of nucleic acid sequences is contained on a substrate.

14. The method of claim 13, wherein the substrate is a microarray, macroarray, Southern blot, slot blot, dot blot, or Northern blot.

15. The method of claim 1, further comprising:
(c) identifying from the plurality of nucleic acid sequences at least one sequence, or a homolog thereof, from the nucleic acid sample obtained from the *Bacillus subtilis* cells cultivated in the presence of the antimicrobial compound that has a detected expression level that is significantly different from the nucleic acid sample obtained from the *Bacillus subtilis* cells cultivated in the absence of the antimicrobial compound.

16. The method of claim 15, wherein the difference in the detected expression level is at least about 10% or greater.

17. The method of claim 15, wherein the difference in the detected expression level is at least about 20% or greater.

18. The method of claim 15, wherein the difference in the detected expression level is at least about 50% or greater.

19. The method of claim 15, wherein the difference in the detected expression level is at least about 75% or greater.

20. The method of claim 15, wherein the difference in the detected expression level is at least about 100% or greater.

21. The method of claim 1, wherein the plurality of nucleic acid sequences is selected from the group of genes of Tables 4-21 or fragments thereof.

22. The method of claim 1, wherein the plurality of nucleic acid sequences is a marker gene for the mode of action of topoisomerase activity inhibition selected from the group of genes in Tables 4, 5, 6 or 7, or fragments thereof.

23. The method of claim 1, wherein the plurality of nucleic acid sequences includes *yerQ* or a fragment thereof.

24. The method of claim 1, wherein the plurality of nucleic acid sequences is a marker for the mode of action of cell wall inhibitors selected from the group of genes of Tables 8, 9, and 10, or fragments thereof.

25. The method of claim 1, wherein the plurality of nucleic acid sequences is a marker for the mode of action of protein synthesis inhibitors selected from the group of genes of Tables 11-20.

26. The method of claim 1, wherein the plurality of nucleic acid sequences is a marker gene for the mode of action of RNA synthesis inhibition selected from the group of genes in Table 21, or fragments thereof.

## Patentansprüche

1. Verfahren zum Bestimmen des Wirkmechanismus einer antimikrobiellen Verbindung, umfassend:
(a) Detektieren von Hybridisierungskomplexen, gebildet durch In-Kontaktbringen von mindestens einer Nukleinsäureprobe, erhalten durch Kultivieren von Bacillus subtilis Zellen in der Gegenwart von mindestens einer subinhibitorischen Menge einer antimikrobiellen Verbindung mit einem unbekannten Wirkmechanismus, mit einer Vielzahl von Nukleinsäuresequenzen, die Genen der Bacillus subtilis Zellen entsprechen, wobei die Gegenwart, Abwesenheit oder Änderung in der Menge der detektierten Hybridisierungskomplexe verglichen mit Hybridisierungskomplexen, gebildet zwischen der Vielzahl von Nukleinsäuresequenzen und einer zweiten Nukleinsäureprobe, erhalten aus den Bacillus subtilis Zellen, kultiviert in der Abwesenheit oder Gegenwart einer Standardverbindung mit einem bekannten Wirkmechanismus, ausgewählt aus der Gruppe bestehend aus Cephalothin, Chloramphenicol, Ciprofloxacin, Erythromycin, Gentamicin, Gramcidin A, Nalidixinsäure, Norfloxacin, Novobiocin, Rifampin, Streptomycin, Trimethoprim und Vancomycin, eine Ähnlichkeit oder Unähnlichkeit der Wirkmechanismen der antimikrobiellen Verbindung und der Standardverbindung anzeigt; und
(b) Zuweisen eines Wirkmechanismus zu der antimikrobiellen Verbindung, basierend auf den den in (a) detektierten Hybridisierungskomplexen zugewiesenen Ähnlichkeits- oder Unähnlichkeitswerten, basierend auf der relativen Menge an Hybridisierung zu einem zweiten Satz von Hybridisierungswerten, die den aus der zweiten Nukleinsäureprobe gebildeten Hybridisierungskomplexen zugewiesen sind;
wobei die sub-inhibitorische Menge auf der minimalen inhibitorischen Konzentration (minimal inhibitory concentration, MIC) der antimikrobiellen Verbindung gegen ein Bakterium und Kultivierung des Bakteriums bei einer oder mehreren Konzentrationen unterhalb der MIC basiert ist, wobei MIC als die Konzentration einer antimikrobiellen Verbindung definiert ist, die nicht in sichtbarem Wachstum des Organismus resultiert.

2. Verfahren nach Anspruch 1, wobei der Schritt in (b) das Unterziehen des Satzes von Werten einem Analyseprogramm umfasst.

3. Verfahren nach Anspruch 2, wobei das Analyseprogramm einen Computeralgorithmus umfasst.

4. Verfahren nach Anspruch 1, wobei die antimikrobielle Verbindung ein Mitglied der Klasse von antimikrobiellen Verbindungen ist, die die Zellwandsynthese inhibiert, die Zellmembran beeinträchtigt, die Proteinsynthese inhibiert, Topoisomeraseaktivität inhibiert, RNA-Synthese inhibiert oder ein kompetetiver Inhibitor ist.

5. Verfahren nach Anspruch 1, wobei die Vielzahl von Sequenzen aus einem sich von Bacillus subtilis unterscheidendem Organismus erhalten werden.

6. Verfahren nach Anspruch 1, wobei die Vielzahl von Sequenzen aus Bacillus subtilis erhalten werden.

7. Verfahren nach Anspruch 1, wobei die Vielzahl von Sequenzen zu weniger als etwa 75% dem Genom von Bacillus subtilis Zellen entsprechen.

8. Verfahren nach Anspruch 1, wobei die Vielzahl von Sequenzen zu weniger als etwa 50% dem Genom von Bacillus subtilis Zellen entsprechen.

9. Verfahren nach Anspruch 1, wobei die Vielzahl von Sequenzen zu weniger als etwa 25% dem Genom von Bacillus subtilis Zellen entsprechen.

10. Verfahren nach Anspruch 1, wobei die Vielzahl von Sequenzen zu weniger als etwa 10% dem Genom von Bacillus subtilis Zellen entsprechen.

11. Verfahren nach Anspruch 1, wobei die Vielzahl von Sequenzen zu weniger als etwa 5% dem Genom von Bacillus subtilis Zellen entsprechen.

12. Verfahren nach Anspruch 1, wobei die Vielzahl von Sequenzen zu weniger als etwa 2% dem Genom von Bacillus subtilis Zellen entsprechen.

13. Verfahren nach Anspruch 1, wobei die Vielzahl von Sequenzen auf einem Substrat enthalten ist.

14. Verfahren nach Anspruch 13, wobei das Substrat ein Mikroarray, ein Makroarray, ein Southern Blot, Slot Blot, Dot Blot oder Northern Blot ist.

15. Verfahren nach Anspruch 1, weiterhin umfassend:
(c) Identifizieren mindestens einer Sequenz aus der Vielzahl von Nukleinsäuresequenzen, oder eines Homologs davon, aus der Nukleinsäureprobe, erhalten aus den Bacillus subtilis Zellen, kultiviert in der Gegenwart der antimikrobiellen Verbindung, die einen detektierten Expressionsspiegel hat, der sich signifikant von der aus den in der Abwesenheit der antimikrobiellen Verbindung kultivierten Bacillus subtilis Zellen erhaltenen Nukleinsäureprobe unterscheidet.

16. Verfahren nach Anspruch 15, wobei der Unterschied in dem detektierten Expressionsspiegel mindestens etwa 10% oder mehr beträgt.

17. Verfahren nach Anspruch 15, wobei der Unterschied in dem detektierten Expressionsspiegel mindestens etwa 20% oder mehr beträgt.

18. Verfahren nach Anspruch 15, wobei der Unterschied in dem detektierten Expressionsspiegel mindestens etwa 50% oder mehr beträgt.

19. Verfahren nach Anspruch 15, wobei der Unterschied in dem detektierten Expressionsspiegel mindestens etwa 75% oder mehr beträgt.

20. Verfahren nach Anspruch 15, wobei der Unterschied in dem detektierten Expressionsspiegel mindestens etwa 100% oder mehr beträgt.

21. Verfahren nach Anspruch 1, wobei die Vielzahl von Nukleinsäuresequenzen ausgewählt ist aus der Gruppe von Genen von Tabellen 4-21 oder Fragmenten davon.

22. Verfahren nach Anspruch 1, wobei die Vielzahl von Nukleinsäuresequenzen ein Markergen für den Wirkmechanismus von Topoisomeraseaktivitätsinhibition ist, ausgewählt aus der Gruppe von Genen der Tabellen 4, 5, 6 oder 7, oder Fragmenten davon.

23. Verfahren nach Anspruch 1, wobei die Vielzahl von Nukleinsäuresequenzen yerQ oder ein Fragment davon einschließt.

24. Verfahren nach Anspruch 1, wobei die Vielzahl von Nukleinsäuresequenzen ein Markergen für den Wirkmechanismus von Zellwandinhibitoren ist, ausgewählt aus der Gruppe von Genen der Tabellen 8, 9 oder 10, oder Fragmenten davon.

25. Verfahren nach Anspruch 1, wobei die Vielzahl von Nukleinsäuresequenzen ein Markergen für den Wirkmechanismus von Proteinsyntheseinhibitoren ist, ausgewählt aus der Gruppe von Genen der Tabellen 11-20.

26. Verfahren nach Anspruch 1, wobei die Vielzahl von Nukleinsäuresequenzen ein Markergen für den Wirkmechanismus von RNA-Syntheseinhibition ist, ausgewählt aus der Gruppe von Genen der Tabelle 21, oder Fragmenten davon.

## Revendications

1. Procédé de détermination du mode d'action d'un composé antimicrobien, comprenant :
(a) la détection des complexes d'hybridation formés par la mise en contact d'au moins un échantillon d'acide nucléique, obtenu par culture de cellules de *Bacillus subtilis* en présence d'au moins une quantité sous-inhibitrice d'un composé antimicrobien ayant un mode d'action inconnu, avec une pluralité de séquences d'acide nucléique correspondant aux gènes des cellules de *Bacillus subtilis,* où la présence, l'absence ou le changement de la quantité des complexes d'hybridation détectée, comparativement aux complexes d'hybridation formés entre la pluralité de séquences d'acide nucléique et un second échantillon d'acide nucléique obtenu à partir des cellules de *Bacillus subtilis* cultivées en l'absence ou en présence d'un composé étalon ayant un mode d'action connu choisi dans le groupe constitué par la céfalotine, le chloramphénicol, la ciprofloxacine, l'érythromycine, la gentamicine, la gramicidine A, l'acide nalidixique, la norfloxacine, la novobiocine, la rifampine, la streptomycine, le triméthoprime et la vancomycine, indique la similarité ou la différence du mode d'actions du composé antimicrobien et du composé étalon ; et
(b) l'attribution d'un mode d'action au composé antimicrobien en se basant sur la similarité ou la différence des valeurs attribuées aux complexes d'hybridation détectés en (a) en se basant sur la quantité relative d'hybridation par rapport à un second ensemble de valeurs d'hybridation attribuées aux complexes d'hybridation formés à partir du second échantillon d'acide nucléique ;
dans lequel la quantité sous-inhibitrice est basée sur la concentration minimale inhibitrice (CMI) du composé antimicrobien contre une bactérie et la culture de la bactérie à une ou plusieurs concentrations inférieures à la CMI, où la CMI est définie comme la concentration d'un composé antimicrobien n'entraînant aucune croissance visible de l'organisme.

2. Procédé selon la revendication 1, dans lequel l'étape en (b) comprend la soumission de l'ensemble des valeurs à un programme pour analyse.

3. Procédé selon la revendication 2, dans lequel le programme pour analyse comprend un algorithme informatique.

4. Procédé selon la revendication 1, dans lequel le composé antimicrobien est un membre de la classe des composés antimicrobiens qui inhibent la synthèse de la paroi cellulaire, interfèrent avec la membrane cellulaire, inhibent la synthèse de protéine, inhibent l'activité topoisomérase, inhibent la synthèse d'ARN, ou est un inhibiteur compétitif.

5. Procédé selon la revendication 1, dans lequel la pluralité de séquences est obtenue à partir d'un organisme différent de *Bacillus subtilis.*

6. Procédé selon la revendication 1, dans lequel la pluralité de séquences est obtenue à partir de *Bacillus subtilis.*

7. Procédé selon la revendication 1, dans lequel la pluralité de séquences correspond à moins d'environ 75 % du génome des cellules de *Bacillus subtilis.*

8. Procédé selon la revendication 1, dans lequel la pluralité de séquences correspond à moins d'environ 50 % du génome des cellules de *Bacillus subtilis.*

9. Procédé selon la revendication 1, dans lequel la pluralité de séquences correspond à moins d'environ 25 % du génome des cellules de *Bacillus subtilis.*

10. Procédé selon la revendication 1, dans lequel la pluralité de séquences correspond à moins d'environ 10 % du génome des cellules de *Bacillus subtilis.*

11. Procédé selon la revendication 1, dans lequel la pluralité de séquences correspond à moins d'environ 5 % du génome des cellules de *Bacillus subtilis.*

12. Procédé selon la revendication 1, dans lequel la pluralité de séquences correspond à moins d'environ 2 % du génome des cellules de *Bacillus subtilis.*

13. Procédé selon la revendication 1, dans lequel la pluralité de séquences d'acide nucléique est contenue sur un substrat.

14. Procédé selon la revendication 13, dans lequel le substrat est une micropuce, une macropuce, un Southem blot, un slot blot, un dot blot ou un Northem blot.

15. Procédé selon la revendication 1, comprenant en outre :
(c) l'identification parmi la pluralité de séquences d'acide nucléique d'au moins une séquence, ou d'un homologue de celle-ci, provenant de l'échantillon d'acide nucléique obtenu à partir des cellules de *Bacillus subtilis* cultivées en présence du composé antimicrobien qui présente un taux d'expression détecté qui est significativement différent de l'échantillon d'acide nucléique obtenu à partir des cellules de *Bacillus subtilis* cultivées en l'absence du composé antimicrobien.

16. Procédé selon la revendication 15, dans lequel la différence du taux d'expression détecté est d'au moins environ 10 % ou supérieure.

17. Procédé selon la revendication 15, dans lequel la différence du taux d'expression détecté est d'au moins environ 20 % ou supérieure.

18. Procédé selon la revendication 15, dans lequel la différence du taux d'expression détecté est d'au moins environ 50 % ou supérieure.

19. Procédé selon la revendication 15, dans lequel la différence du taux d'expression détecté est d'au moins environ 75 % ou supérieure.

20. Procédé selon la revendication 15, dans lequel la différence du taux d'expression détecté est d'au moins environ 100 % ou supérieure.

21. Procédé selon la revendication 1, dans lequel la pluralité de séquences d'acide nucléique est choisie dans le groupe de gènes des tableaux 4 à 21 ou des fragments de ceux-ci.

22. Procédé selon la revendication 1, dans lequel la pluralité de séquences d'acide nucléique est un gène marqueur pour le mode d'action de l'inhibition de l'activité topoisomérase choisi dans le groupe de gènes des tableaux 4, 5, 6 ou 7, ou des fragments de ceux-ci.

23. Procédé selon la revendication 1, dans lequel la pluralité de séquences d'acide nucléique comprend *yerQ* ou un fragment de celui-ci.

24. Procédé selon la revendication 1, dans lequel la pluralité de séquences d'acide nucléique est un marqueur pour le mode d'action des inhibiteurs de la paroi cellulaire choisi dans le groupe des gènes des tableaux 8, 9 et 10, ou des fragments de ceux-ci.

25. Procédé selon la revendication 1, dans lequel la pluralité de séquences d'acide nucléique est un marqueur pour le mode d'action des inhibiteurs de la synthèse de protéine choisi dans le groupe des gènes des tableaux 11 à 20.

26. Procédé selon la revendication 1, dans lequel la pluralité de séquences d'acide nucléique est un gène marqueur pour le mode d'action de l'inhibition de la synthèse d'ARN choisi dans le groupe des gènes du tableau 21, ou des fragments de ceux-ci.
